(19) Europäisches Patentamt
European Patent Office
Office européen des brevets

(11) EP 4 467 544 A1

(12) **EUROPEAN PATENT APPLICATION**
published in accordance with Art. 153(4) EPC

(43) Date of publication:
**27.11.2024 Bulletin 2024/48**

(21) Application number: **23743009.5**

(22) Date of filing: **28.01.2023**

(51) International Patent Classification (IPC):
***C07D 471/04*** (2006.01) ***C07D 247/02*** (2006.01)
***C07D 309/16*** (2006.01) ***A61K 31/437*** (2006.01)
***A61K 31/506*** (2006.01) ***A61K 31/341*** (2006.01)
***A61P 19/10*** (2006.01) ***A61P 35/00*** (2006.01)
***A61P 9/00*** (2006.01)

(52) Cooperative Patent Classification (CPC):
**A61K 31/341; A61K 31/437; A61K 31/506; A61P 9/00; A61P 19/10; A61P 35/00; C07D 247/02; C07D 309/16; C07D 471/04**

(86) International application number:
**PCT/CN2023/073612**

(87) International publication number:
**WO 2023/138695 (27.07.2023 Gazette 2023/30)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **21.01.2022 CN 202210098531**
**21.02.2022 CN 202210156862**

(71) Applicant: **Shandong New Time Pharmaceutical Co., Ltd.**
**Linyi City, Shandong 273400 (CN)**

(72) Inventors:
- **WANG, Jinxin**
  **Nanjing, Jiangsu 210009 (CN)**
- **ZHANG, Guimin**
  **Linyi, Shandong 276006 (CN)**
- **WANG, Ke**
  **Nanjing, Jiangsu 210009 (CN)**
- **YAO, Jingchun**
  **Linyi, Shandong 276006 (CN)**
- **PAN, Lihong**
  **Linyi, Shandong 276006 (CN)**

(74) Representative: **J A Kemp LLP**
**80 Turnmill Street**
**London EC1M 5QU (GB)**

# (54) CATHEPSIN K INHIBITOR, AND PREPARATION METHOD THEREFOR AND USE THEREOF

(57) The present invention relates to a cathepsin K inhibitor having the structure as shown in formula (0) and/or formula (II), wherein cyanopyrimidine or a keto group as an electrophilic group is a key group to exert a good inhibitory effect on cathepsin K. Further provided are a preparation method therefor and use thereof. The provided cathepsin K inhibitor has a relatively high inhibitory effect and selectivity, and is expected to be used for treating diseases including thyroid diseases, cardiovascular diseases, bone diseases and gum diseases.

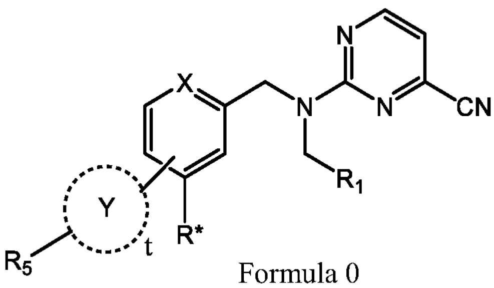

Formula 0

or

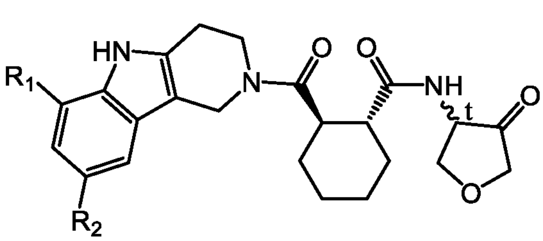

Formula II

.

EP 4 467 544 A1

## Description

### TECHNICAL FIELD

The present invention relates to the field of medicinal chemistry, specifically to a compound inhibiting the cathepsin K activity, a method for preparing same and use thereof in the preparation of a drug for treating a disease related with the cathepsin K activity or characterized by the cathepsin K activity, such as osteoporosis.

### BACKGROUND ART

Osteoporosis is a metabolic disorder that reduces the bone mass of the whole body and bone density, and further increases the fracture risk of patients. With the aggravation of aging of population, the health problems related to the disease become more serious day by day and the fracture probability of the patients with osteoporosis is greatly increased, thereby bringing heavy burden to society and families and seriously influencing the life of people. The pathogenesis of the osteoporosis is caused by imbalance between osteoclast-mediated bone resorption and osteoblast-mediated bone formation. Common drugs for treating osteoporosis in the market at present comprise a bone resorption inhibitor such as diphosphate and a bone formation promoter, but both drugs have certain defects. The long-term use of the bone resorption inhibitor can influence the differentiation and proliferation of osteocytes, and further causes the occurrence of a low bone turnover state; the bone formation promoter increases the probability of osteosarcoma in the patients.

Since the drugs in the market at present all have certain defects, it is a current research direction on researching a novel drug for treating osteoporosis, which can treat the osteoporosis, does not influence the proliferation and differentiation of osteoblasts and osteoclasts, and avoids the occurrence of the low bone turnover state.

Cathepsin K (Cat K) belongs to a cysteine protease of the papain family, is abundant in osteoclasts, and becomes a new target for treating osteoporosis currently. The human bone matrix consists of 25% of water, 25% of an organic matrix and 50% of a mineral matrix, wherein 90% of the composition of the organic matrix is type I collagen. The degradation of the type I collagen is a key process of osteoclast-mediated bone resorption. The Cat K plays a leading role in the degradation of the type I collagen. A Cat K inhibitor inhibits the resorption of mature osteoclasts by blocking the degradation of matrix collagen, and simultaneously can maintain the survival number of osteoclasts, such that coupling signals of the osteoclasts and osteoblasts are kept intact, and the occurrence of the low bone turnover state is avoided. In addition, the Cat K plays an important role in the pathogenic process of thyroid diseases, cardiovascular diseases and gum diseases. Diseases characterized by abnormal expression or activation of the Cat K comprise thyroid diseases, cardiovascular diseases, bone diseases and gum diseases, specifically hyperthyroidism, atherosclerosis, cardiac hypertrophy, heart failure, osteoporosis, osteoarthritis, rheumatoid arthritis, gingivitis and periodontitis. In recent years, there are more and more researches on the Cat K. The Cat K secretion of endothelial cells can be increased under pathological conditions such as coronary atherosclerosis. The Cat K is closely related to the occurrence and development of myocardial hypertrophy and heart failure. Garg et al. (Garg G, Pradeep AR, Thorat MK, et al. Effect of nonsurgical periodontal therapy on crevicular fluid levels of cathepsin K in periodontitis. Arch Oral Biol, 54: 1046-1051) proved that after the basic treatment, along with the reduction of the clinical gingival index, the periodontal probing depth and the attachment loss of patients with periodontitis, the level of Cat K was reduced through change of the Cat K in a gingival crevicular fluid before and after the basic treatment of the patients with periodontitis. The Cat K is used as a marker of periodontitis bone absorption and needs to be given further attention and research in the periodontitis treatment..

Currently, the activity of Cat K inhibitors and various inhibitors reported in a clinical research stage are all greatly improved. However, no drug targeting this target is available on the market. The main reason is that a compound has poor selectivity and relatively high inhibitory activity against other subtypes such as B-type and S-type, resulting in the occurrence of side effects of the drug. Therefore, it is a main research direction at present in the development and research of a novel Cat K inhibitor with high efficiency and selectivity aiming at reducing side effects.

Since Cat B and Cat K belong to cysteine proteases, but Cat L is an eosinophilic protease and is difficult to obtain, the present invention designs and synthesizes a cyano-substituted pyrimidine compound, and the activities thereof against K, B and S are determined. It is found that the compound has good selectivity and thus is expected to be used for treating diseases related to the Cat K activity or characterized by the Cat K activity.

### SUMMARY

The present invention provides a novel ketone compound and/or a nitrile compound acting on Cat K and a pharmaceutically acceptable salt thereof, and a preparation method therefor and use thereof.

a) The present invention provides a nitrile compound whose structural general formula is shown as follows:

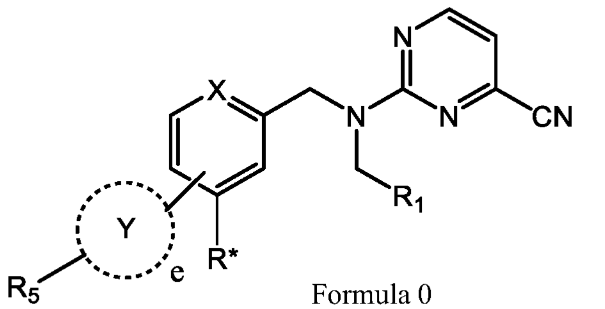

Formula 0

wherein in formula 0, X is C or N;
$R_1$ is selected from H, substituted or unsubstituted C1-10 alkyl, and substituted or unsubstituted C3-C10 cycloalkyl, wherein the substituent is selected from halogen, amino, cyano, hydroxyl, an aldehyde group, carboxyl and sulfonyl;
R* is selected from halogen or C1-6 alkyl;
Y is a ring group, located at any position of an attached aromatic ring, attached to the aromatic ring through 1 or 2 carbon atoms, and optionally C3-10 cycloalkyl, a C6-12 aromatic ring or a C5-12 heterocyclic ring;
wherein the C6-12 aromatic ring contains a C6-12 aromatic ring or a C6-12 heteroaromatic ring; wherein the C6-12 heteroaromatic ring contains at least one heteroatom; wherein the C5-12 heterocyclic ring is a saturated heterocyclic ring or an unsaturated heterocyclic ring, and the heterocyclic ring contains 1-3 heteroatoms; wherein the heteroatom is optionally O, N or S;
e is 0 or 1; when e is 0, $R_5$ is directly attached to any position of the aromatic ring; and
$R_5$ is selected from H, halogen, amino, cyano, C1-10 alkyl, C1-10 alkoxy, C3-10 cycloalkyl, substituted or unsubstituted C3-10 heterocycloalkyl, -S(O) $2R_2$, $-C(O)R_2$, $-NR_3R_4$, $-C(O)NHR_7$, $-SR_6$ and $-OR_6$;
wherein the substituent of the C3-10 heterocycloalkyl is selected from hydroxyl and C1-10 alkyl;
wherein the C1-10 alkyl may be further substituted by hydroxy or $-C(O)R_2$;
wherein $R_2$ is selected from H, amino, halogen, substituted or unsubstituted C1-6 alkyl, C1-6 alkoxy, C3-8 cycloalkyl and C3-8 heterocycloalkyl, wherein the substituent of the C1-6 alkyl, the C1-6 alkoxy, the C3-8 cycloalkyl or the C3-8 heterocycloalkyl is C1-6 alkyl;
wherein $R_3$ and $R_4$ are each independently selected from H and $-C(O)R_8$; or $R_3$ and $R_4$ together with N to which they are attached form a 4-8 membered ring containing at least one N; wherein $R_8$ is selected from C1-6 alkyl substituted by piperazinyl or methylpiperazinyl;
wherein $R_6$ is selected from C1-6 alkyl;
wherein $R_7$ is selected from C1-6 alkyl, wherein the C1-6 alkyl may be further substituted by $-C(O)R_9$; wherein $R_9$ is selected from piperazinyl or methylpiperazinyl;
wherein the C3-10 heterocycloalkyl or the C3-8 heterocycloalkyl contains 1-3 heteroatoms, and the heteroatom is optionally O, N or S; and
wherein the halogen is mono-substituted or poly-substituted and selected from F, Cl, Br and I.

When t is 0 and R* is H, the compound of formula 0 is as shown in formula I:

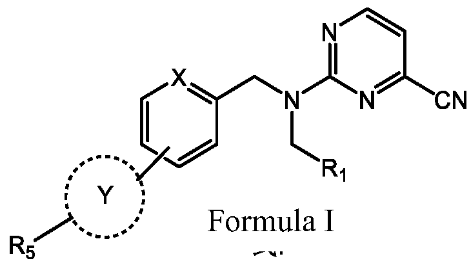

Formula I

wherein in formula I, X is C or N;
$R_1$ is selected from H, substituted or unsubstituted C1-10 alkyl, and substituted or unsubstituted C3-C10 cycloalkyl; wherein the substituent is selected from halogen, amino, cyano, hydroxyl, an aldehyde group, carboxyl and sulfonyl;
Y is a ring group, located at any position of an attached aromatic ring, and optionally C3-10 cycloalkyl, a C6-12 aromatic ring or a C5-12 heterocyclic ring,
wherein the C6-12 aromatic ring contains a C6-12 aromatic ring or a C6-12 heteroaromatic ring; wherein the C6-12

heteroaromatic ring contains at least one heteroatom; wherein the C5-12 heterocyclic ring is a saturated heterocyclic ring or an unsaturated heterocyclic ring, and the heterocyclic ring contains 1-3 heteroatoms; wherein the heteroatom is optionally O, N or S;

$R_5$ is selected from H, halogen, amino, cyano, C1-10 alkyl, C1-10 alkoxy, C3-10 cycloalkyl, -S(O) $2R_2$, -C(O)$R_2$, -$NR_3R_4$, -$SR_6$ and -$OR_6$; wherein $R_2$ is selected from H, amino, halogen, C1-6 alkyl, C1-6 alkoxy and C3-8 cycloalkyl; wherein $R_3$ and $R_4$ together with N to which they are attached form a 4-8 membered ring containing at least one N; wherein $R_6$ is selected from C1-6 alkyl; and

wherein the halogen is mono-substituted or poly-substituted and selected from F, Cl, Br and I.

Further, X is C;

$R_1$ is selected from C1-6 alkyl and C4-C8 cycloalkyl;

Y is a ring group, located at a para-position of an aromatic ring, and optionally C4-8 cycloalkyl, a C6-10 aromatic ring or a C5-10 heterocyclic ring,

wherein the C6-10 aromatic ring contains a C6-10 aromatic ring or a C6-10 heteroaromatic ring; wherein the C6-10 heteroaromatic ring contains at least one heteroatom; wherein the C5-10 heterocyclic ring is a saturated heterocyclic ring or an unsaturated heterocyclic ring, and the heterocyclic ring contains 1-3 heteroatoms; wherein the heteroatom is optionally O, N or S; and

$R_5$ is selected from H, halogen, amino, cyano, C1-6 alkyl, C1-6 alkoxy, C3-8 cycloalkyl, -S(O) $2R_2$, -C(O)$R_2$, -$NR_3R_4$, -$SR_6$ and -$OR_6$; wherein $R_2$ is selected from H, amino, halogen, C1-6 alkyl, C1-6 alkoxy and C3-8 cycloalkyl; wherein $R_3$ and $R_4$ together with N to which they are attached form a 5-8 membered ring containing at least one N; wherein $R_6$ is selected from C1-6 alkyl; and

wherein the halogen is mono-substituted or poly-substituted and selected from F, Cl and Br.

Further, X is C;

$R_1$ is selected from C1-6 alkyl and C5-8 cycloalkyl;

Y is a ring group, located at a para-position of an aromatic ring, and optionally C5-8 cycloalkyl, a C6-8 aromatic ring or a C5-8 heterocyclic ring,

wherein the C6-8 aromatic ring contains a C6-8 aromatic ring or a C6-8 heteroaromatic ring; wherein the C6-8 heteroaromatic ring contains at least one heteroatom; wherein the C5-8 heterocyclic ring is a saturated heterocyclic ring or an unsaturated heterocyclic ring, and the heterocyclic ring contains 1-3 heteroatoms; wherein the heteroatom is optionally O, N or S; and

$R_5$ is selected from H, halogen, amino, cyano, C1-3 alkyl, C1-3 alkoxy, -S(O) $2R_2$, - C(O)$R_2$, -$NR_3R_4$, -$SR_6$ and -$OR_6$; wherein $R_2$ is selected from amino, halogen and C1-3 alkyl; wherein $R_3$ and $R_4$ together with N to which they are attached form a 5-8 membered ring containing at least one N; wherein $R_6$ is selected from C1-3 alkyl; and

wherein the halogen is mono-substituted or poly-substituted and selected from F, Cl and Br.

In some examples, $R_1$ is selected from neopentyl and cyclohexyl.

In some examples, Y is selected from the following groups:

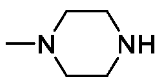

,

phenyl, pyridyl, thiophenyl and thiazolyl.

In some preferred examples, Y is selected from

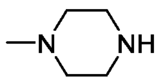

,

phenyl and thiazolyl.

In some examples, $R_5$ is selected from H, F, Cl, cyano, methyl, methylthio, methoxy, methylsulfonyl, methylcarbonyl and methylpiperazinyl.

In some preferred examples, $R_5$ is selected from methyl and methylpiperazinyl.

The present invention provides a ketone cathepsin K inhibitor whose structural general formula is shown as follows:

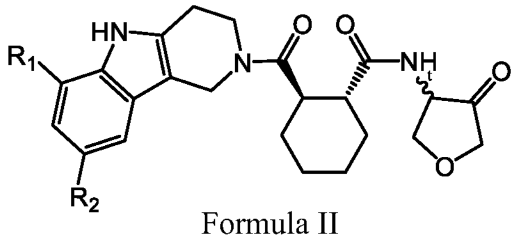

Formula II

wherein $R_1$ and $R_2$ are each independently selected from H, halogen, cyano, amino, substituted or unsubstituted C1-6 alkyl, and substituted or unsubstituted C1-6 alkoxy, and the substituted C1-6 alkyl or substituted C1-6 alkoxy is further substituted by at least one halogen or hydroxyl; and

t is a chemical bond, optionally at least one of 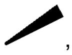, 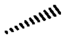 or 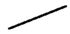.

Further, $R_1$ and $R_2$ are each independently selected from H, halogen, C1-3 alkyl, and C1-3 alkoxy, and the C1-3 alkyl or C1-3 alkoxy is further substituted by at least one halogen.

Further, $R_1$ and $R_2$ are each independently selected from H, halogen, C1-3 alkyl and C1-3 alkoxy, and the C1-3 alkyl or C1-3 alkoxy is further substituted by at least one F.

The C1-3 alkyl is methyl, ethyl, propyl or isopropyl.

The C1-3 alkoxy is methoxy, ethoxy, propoxy, isopropoxy, monofluoromethoxy, difluoromethoxy or trifluoromethoxy.

The C1-3 alkoxy is methoxy or trifluoromethyl.

The C1-3 alkoxy is trifluoromethyl.

In some examples, $R_1$ and $R_2$ are each independently selected from H, F, Cl, Br, methoxy and trifluoromethyl.

In some preferred examples, $R_1$ and $R_2$ are each independently selected from H and F.

The halogen is selected from F, Cl, Br and I.

The compound provided by the present invention comprises the following specific structures:

1) 2-[(2,2-dimethylpropyl){[4-(4-methylpiperazin-1-yl)phenyl]methyl}amino]pyrimidine-4-carbonitrile;
2) 2-[(2,2-dimethylpropyl)[(4-phenylphenyl)methyl]amino]pyrimidine-4-carbonitrile;
3) 2-[(2,2-dimethylpropyl) ({4-[4-(methylthio)phenyl]phenyl} methyl)amino]pyrimidine-4-carbonitrile;
4) 2-[(2,2-dimethylpropyl){[4-(4-fluorophenyl)phenyl]methyl}amino]pyrimidine-4-carbonitrile;
5) 2-[(2,2-dimethylpropyl){[4-(4-methoxyphenyl)phenyl]methyl}amino]pyrimidine-4-carbonitrile;
6) 2-[(2,2-dimethylpropyl) ({4-[4-(methylsulfonyl)phenyl]phenyl}methyl)amino]pyrimidine-4-carbonitrile;
7) 2-({[4-(5-cyanothiophen-2-yl)phenyl]methyl} (2,2dimethylpropyl)amino)pyrimidine-4-carbonitrile;
8) 2-({[4-(6-chloropyridin-3-yl)phenyl]methyl}(2,2-dimethylpropyl)amino)pyrimidine-4-carbonitrile;
9) 2-({[4-(3,4-dichlorophenyl)phenyl]methyl}(2,2-dimethylpropyl)amino)pyrimidine-4-carbonitrile;
10) 2-[(2,2-dimethylpropyl){[4-(5-methylthiophen-2-yl)phenyl]methyl}amino]pyrimidine-4-carbonitrile;
11) 4-(4-{[(4-cyanopyrimidin-2-yl) (2,2-dimethylpropyl)amino]methyl}phenyl)methyl benzoate;
12) 2-({[4-(4-chloro-3-fluorophenyl)phenyl]methyl}(2,2-dimethylpropyl)amino)pyrimidine-4-carbonitrile;
13) 2-[(2,2-dimethylpropyl) ({4-[4-(4-methylpiperazin-1-yl)phenyl]phenyl} methyl)amino]pyrimidine-4-carbonitrile;
14) 2-[(cyclohexylmethyl)[(4-phenylphenyl)methyl]amino]pyrimidine-4-carbonitrile;
15) 2-[(cyclohexylmethyl) ({4-[4-(4-methylpiperazin-1-yl)phenyl]phenyl} methyl)amino]pyrimidine-4-carbonitrile;
16) 2-[(cyclohexylmethyl) ({4-[4-(methylsulfonyl)phenyl]phenyl}methyl)amino]pyrimidine-4-carbonitrile;
17) 2-(neopentyl(4-(thiazol-4-yl)benzyl)amino)pyrimidine-4-carbonitrile;
18) 2-((4-(2-(4-methylpiperazin-1-yl)thiazol-4-yl)benzyl) (neopentyl)amino)pyrimidine-4-carbonitrile
19) (1R,2R)-2-(8-fluoro-2,3,4,9-tetrahydro-1H-pyrido[3,4-b]indole-2-carbonyl)-N-(4-oxotetrahydrofuran-3-yl)cyclohexane-1-carboxamide;
20) (1R,2R)-2-(2,3,4,5-tetrahydro-1H-pyrido[4,3-b]indole-2-carbonyl)-N-(4-oxotetrahydrofuran-3-yl)-cyclohexane-1 -carboxamide;
21) (1R,2R)-2-(8-fluoro-2,3,4,9-tetrahydro-1H-pyrido[3,4-b]indole-2-carbonyl)-N-((S)-4-oxotetrahydrofuran-3-yl)cyclohexane-1-carboxamide;
22) (1R,2R)-2-(6,8-difluoro-2,3,4,9-tetrahydro-1H-pyrido[3,4-b]indole-2-carbonyl)-N-((S)-4-oxotetrahydrofuran-3-yl)cyclohexane-1-carboxamide;
23) (1R,2R)-2-(8-chloro-2,3,4,5-tetrahydro-1H-pyrido[4,3-b]indole-2-carbonyl)-N-((S)-4-oxotetrahydrofuran-3-yl) cyclohexane-1-carboxamide;
24) (1R,2R)-2-(8-bromo-2,3,4,5-tetrahydro-1H-pyrido[4,3-b]indole-2-carbonyl)-N-((S)-4-oxotetrahydrofuran-3-yl)cyclohexane-1-carboxamide;

25) (1R,2R)-2-(6-(trifluoromethoxy)-2,3,4,5-tetrahydro-1H-pyrido[4,3-b]indole-2-carbonyl)-N-(4-oxotetrahydrofuran-3-yl)-cyclohexane-1-carboxamide;

26) (1R,2R)-2-(6-(trifluoromethoxy)-2,3,4,5-tetrahydro-1H-pyrido[4,3-b]indole-2-carbonyl)-N-(S)-(4-oxotetrahydrofuran-3-yl)-cyclohexane-1-carboxamide;

27) (1R,2R)-2-(6-fluoro-2,3,4,5-tetrahydro-1H-pyrido[4,3-b]indole-2-carbonyl)-N-(S)-(4-oxotetrahydrofuran-3-yl)-cyclohexane-1-carboxamide;

28) (1R,2R)-2-(6-fluoro-2,3,4,5-tetrahydro-1H-pyrido[4,3-b]indole-2-carbonyl)-N-(S)-(4-oxotetrahydrofuran-3-yl)-cyclohexane-1-carboxamide;

29) (1R,2R)-2-(6-methoxy-2,3,4,5-tetrahydro-1H-pyrido[4,3-b]indole-2-carbonyl)-N-(S)-(4-oxotetrahydrofuran-3-yl)-cyclohexane-1-carboxamide;

30) (1R,2R)-2-(6-methoxy-2,3,4,5-tetrahydro-1H-pyrido[4,3-b]indole-2-carbonyl)-N-(4-oxotetrahydrofuran-3-yl)-cyclohexane-1-carboxamide;

31) (1R,2R)-2-(8-fluoro-6-methoxy-2,3,4,5-tetrahydro-1H-pyrido[4,3-b]indole-2-carbonyl)-N-(S)-(4-oxotetrahydrofuran-3-yl)-cyclohexane-1-carboxamide;

32) (1R,2R)-2-(8-fluoro-6-methoxy-2,3,4,5-tetrahydro-1H-pyrido[4,3-b]indole-2-carbonyl)-N-(4-oxotetrahydrofuran-3-yl)-cyclohexane-1-carboxamide;

33) 2-((4-chlorobenzyl) (neopentyl)amino)pyrimidine-4-carbonitrile;

34) 2-((4-bromobenzyl) (neopentyl)amino)pyrimidine-4-carbonitrile;

35) 2-((naphthalen-2-ylmethyl) (neopentyl)amino)pyrimidine-4-carbonitrile;

36) 2-(neopentyl(quinolin-2-ylmethyl)amino)pyrimidine-4-carbonitrile;

37) 2-(((4'-(4-(2-hydroxyethyl)piperazin-1-yl)-[1,1'-biphenyl]-4-yl)methyl) (neopentyl)amino)pyrimidine-4-carbonitrile;

38) 2-(((4'-(4-hydroxypiperidin-1-yl)-[1,1'-biphenyl]-4-yl)methyl) (neopentyl)amino)pyrimidine-4-carbonitrile;

39) 2-(((2-methyl-4'-(4-methylpiperazin-1-yl)-[1,1'-biphenyl]-4-yl)methyl) (neopentyl)amino)pyrimidine-4-carbonitrile;

40) 2-(((2-chloro-4'-(4-methylpiperazin-1-yl)-[1,1'-biphenyl]-4-yl)methyl) (neopentyl)amino)pyrimidine-4-carbonitrile;

41) 2-(neopentyl(4-(4-(2-oxopropyl)piperazin-1-yl)benzyl)amino)pyrimidine-4-carbonitrile;

42) 2-(isobutyl((4'-(4-methylpiperazin-1-yl)-[1,1'-biphenyl]-4-yl)methyl)amino)pyrimidine-4-carbonitrile;

43) N-(4-((4-cyanopyrimidin-2-yl) (neopentyl)amino)methyl)phenyl)-2-(4-methylpiperazin-1-yl)acetamide;

44) 2-((4-(4-methylpiperazin-1-carbonyl)benzyl) (neopentyl)amino)pyrimidine-4-carbonitrile; and

45) 4-(((4-cyanopyrimidin-2-yl) (neopentyl)amino)methyl)-N-(2-(4-methylpiperazin-1-yl)-2-oxoethyl)benzamide.

Another objective of the present invention is to provide a method for preparing the compound and a pharmaceutically acceptable salt thereof. A method for preparing the compound of formula I comprises the following steps:

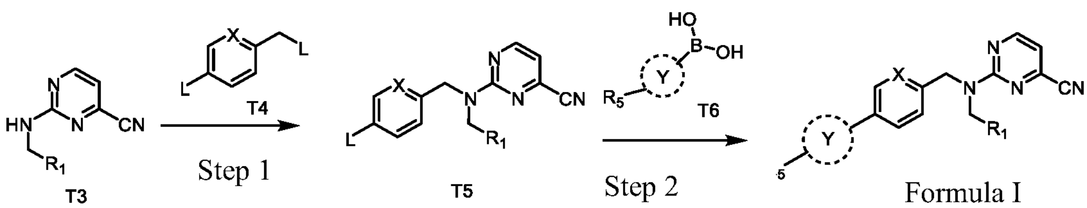


L is halogen

wherein $R_1$, $R_5$, X and Y are as defined above;

wherein L is halogen and selected from F, Cl and Br;

step 1), reacting an intermediate T3 with a compound T4 to generate a compound T5;

reaction condition: in an alkaline solvent;

wherein an alkali is an inorganic alkali and selected from at least one of cesium carbonate, sodium carbonate, potassium carbonate, sodium bicarbonate, potassium bicarbonate, ammonium hydroxide, sodium hydride, sodium hydroxide, potassium hydroxide and calcium hydroxide; and

the solvent is at least one selected from water, methanol, ethanol, glycerol, propylene glycol, formamide, acetonitrile, n-butanol, dioxane, dichloromethane, chloroform, acetone, dimethyl sulfoxide, dimethylformamide, ethyl acetate and tetrahydrofuran; and

**step** 2), reacting a compound T5 with a compound T6 to generate the compound of formula I;

reaction condition: performing heating reaction in an alkaline solvent;
wherein the alkali and the solvent are as defined in step 1); and
wherein the heating is performed at temperature of 40-100°C.

A method for preparing the compound of formula II comprises the following steps:

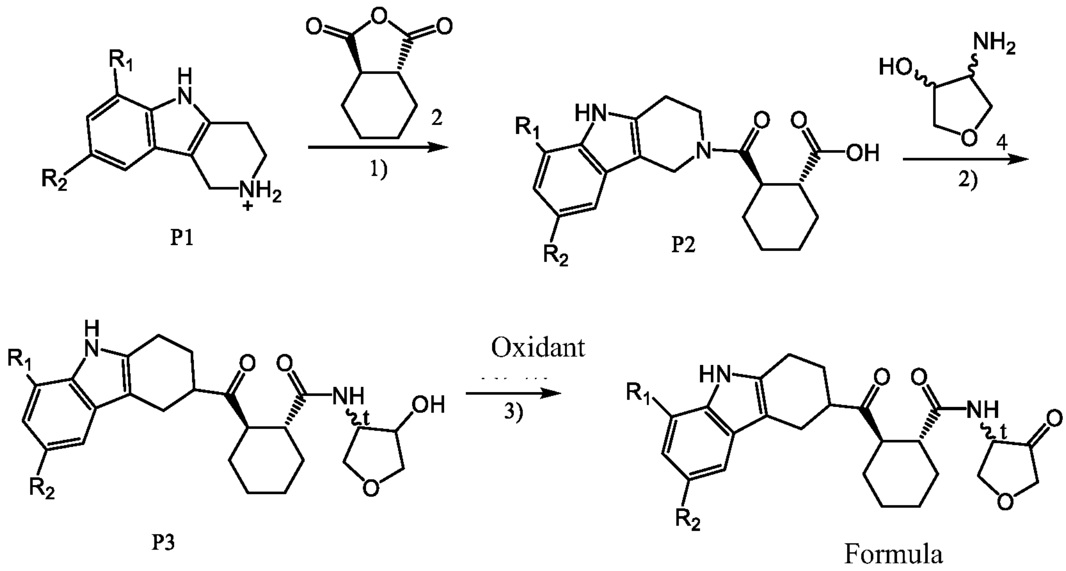


P1 P2 P3 Formula

step 1), reacting a compound P1 with (3aR,7aS)-hexahydroisobenzofuran-1,3-dione to generate a compound P2; and
reaction condition: in a solvent; and the solvent is a polar solvent and selected from at least one of water, methanol, ethanol, glycerol, propylene glycol, formamide, acetonitrile, n-butanol, dioxane, dichloromethane, acetone, dimethyl sulfoxide, dimethylformamide, ethyl acetate and tetrahydrofuran;
**step 2)**, reacting the compound P2 with 4-aminotetrahydrofuran-3-ol to generate a compound P3;
reaction condition: in a polar solvent containing an alkali, wherein the alkali is at least one selected from pyridine, 2-methylpyridine, 2,6-dimethylpyridine, 4-dimethylaminopyridine, triethylamine, diethylamine, N,N-diisopropylethylamine, dimethylisopropylamine, piperidine, 1-methylpiperidine and 1-methylpyrrolidine; and the polar solvent is as defined in step 1); and
**step** 3), oxidizing the compound P3 to generate the compound of formula I;
reaction condition: under the condition of an oxidant; and the oxidant is an inorganic oxidant, an organic oxidant or a mixed oxidant;
wherein the inorganic oxidant is selected from at least one of sodium dichromate, chromic acid, manganese dioxide, ammonium ceric nitrate, potassium permanganate, potassium ferrate, a bromine simple substance, an iodine simple substance, sodium hypochlorite, sodium chlorite, sodium bromate, sodium periodate and iodine pentoxide; the organic oxidant is selected from at least one of 2-iodoxybenzoic acid, tetramethyl piperidine oxide, benzoyl peroxide and cyclohexanone peroxide; and the mixed oxidant is at least one selected from pyridinium chlorochromate, a pyridine chromic anhydride complex, pyridine dichromate and sulfur trioxide pyridine complex; and
further, the oxidant is preferably the pyridinium chlorochromate, the pyridine chromic anhydride complex and the sulfur trioxide pyridine complex.

The present invention further provides a composition containing the cathepsin K inhibitor, a therapeutically effective amount of one or more of the compounds or a pharmaceutically acceptable salt thereof.
The present invention further provides use of the cathepsin K inhibitor in the preparation of a drug for treating a disease taking cathepsin K as a target.
Further, the disease taking cathepsin K as a target comprises a thyroid disease, a cardiovascular disease, a bone disease, a gum disease and a tumor.
Further, the thyroid disease comprises hyperthyroidism.
Further, the cardiovascular disease comprises atherosclerosis, cardiac hypertrophy and heart failure.
Further, the bone disease comprises osteoporosis, osteoarthritis and rheumatoid arthritis.
Further, the gum disease comprises gingivitis and periodontitis.
Further, the tumor comprises tumor invasion and tumor metastasis.

Further, the disease taking cathepsin K as a target is osteoporosis.

Compared with the prior art, the present invention has the following advantages and technical effects.

The compound of the present invention has a high inhibition rate on Cat K, a low $IC_{50}$ value and the good inhibitory activity on the Cat K. The inhibition rate of the compound on the Cat K at 10 μM can reach 90% or more and the $IC_{50}$ is less than 50 nM.

The compound of the present invention has good selectivity: at the levels of 1 μM and 10 μM, the inhibition rates of the compound on Cat B and Cat S are both < 50%, and the $IC_{50}$ values of the compounds on the Cat B and Cat S are both > 10 μM, indicating that the compound of the present invention has good selectivity on the Cat K.

The compound of the present invention has good druggability.

The cathepsin K inhibitor can be used for treating diseases characterized by abnormal expression or activation of the Cat K. The diseases comprise thyroid diseases, cardiovascular diseases, bone diseases and oral diseases, specifically hyperthyroidism, atherosclerosis, cardiac hypertrophy, heart failure, osteoporosis, osteoarthritis, rheumatoid arthritis, gingivitis and periodontitis. The compound of the present invention can inhibit the formation of osteoclasts and can be used for treating osteoporosis.

The terms in the present invention:

1. Abbreviations and definitions

DMF: N,N'-dimethylformamide
DMSO: dimethyl sulfoxide
DMAP: 4-dimethylaminopyridine
DCM: dichloromethane
HATU: 2-(7-azabenzotriazole)-N,N,N',N'-tetramethyluronium hexafluorophosphate
DIPEA: N,N-diisopropyl ethylamine

2. Other terms

The term "C1-10 alkyl" refers to a straight or branched chain hydrocarbyl group containing 1 to 10 carbon atoms derived by the removal of one hydrogen atom from a single carbon atom of a parent alkane, for example, methyl, ethyl, n-propyl, isopropyl, n-butyl, isobutyl, tert-butyl, sec-butyl, n-pentyl, neopentyl, n-hexyl and the like. The term "C1-6 alkyl" refers to any straight or branched chain group containing 1-6 carbon atoms.

The term "C1-10 alkoxy" comprises -O-C1-10 alkyl, referring that the C1-10 alkyl is attached to an oxygen atom.

The term "C1-6 alkoxy" comprises -O-C1-10 alkyl.

The term "C3-10 cycloalkyl" refers to a stable non-aromatic monocyclic or multicyclic hydrocarbon group consisting of only carbon and hydrogen atoms, may include fused, bridged or spiro ring systems, having 3 to 10 carbon atoms, and is saturated or unsaturated and may be attached to the rest of the molecule by a single bond via any suitable carbon atom. Unless otherwise specifically indicated in the description, a carbon atom in the cycloalkyl may be optionally oxidized. An example of the cycloalkyl includes but is not limited to cyclopropyl, cyclobutyl, cyclopentyl, cyclohexyl, cycloheptyl and cyclooctyl.

The term "C6-12 membered aromatic ring" indicates a planar ring system with conjugation, has the bonding between atoms being not a discontinuous single-double bond alternation, but covered by a delocalized π electron cloud, and has 6-12 atoms, which may be carbon, nitrogen, sulfur and oxygen atoms. Unless otherwise specifically indicated in the description, the aromatic ring may be a monocyclic, bicyclic, tricyclic, or more ring system.

The expressions of the terms "C1-10" and "C1-C10" indicate groups containing 1-10 carbon atoms.

The term "C6-12 heteroaromatic ring" is a monocyclic or bicyclic aromatic ring containing 6-12 ring atoms, wherein 1, 2, 3 or 4 ring atoms are selected from nitrogen, sulfur or oxygen, wherein the nitrogen or the sulfur in the ring may be oxidized. For the purpose of the present invention, heteroaryl is preferably a stable 5- to 10-membered aromatic group containing 1 to 3 heteroatoms selected from nitrogen, oxygen and sulfur, more preferably, a stable 5- to 8-membered aromatic group containing 1 to 2 heteroatoms selected from nitrogen, oxygen and sulfur. An example of the heteroaryl includes but is not limited to thienyl, imidazolyl, pyrazolyl, thiazolyl, oxazolyl, oxadiazolyl, isoxazolyl, pyridyl, pyrimidinyl, pyrazinyl and pyridazinyl.

The term "C5-12 heterocyclic ring" is a saturated, unsaturated, or partially saturated monocyclic or bicyclic ring containing 5-12 ring atoms, wherein 1, 2 or 3 ring atoms are selected from nitrogen, sulfur or oxygen and the ring may be attached by carbon or nitrogen, wherein a -CH2- group in the ring is optionally substituted by a -C(O) group, wherein the nitrogen or sulfur atoms in the ring may be optionally oxidized to form a N-oxide or a S-oxide, and wherein -NH- in the ring is optionally substituted by acetyl, formyl, methyl or methanesulfonyl.

Unless defined otherwise, all technical and scientific terms used herein have the same meaning as commonly understood by one of ordinary skills belonging to the art of the subject matter of the claims.

It is to understanding understood that both the foregoing brief description and the following detailed description are exemplary and explanatory only and are not restrictive of the subject matter of the present invention. Furthermore, the term "comprises" and other forms thereof, such as "contains", "includes" and "including" are non-limiting.

The section headings used herein are for organizational purposes only and should not be construed as limiting the described subject matter. All documents or document portions cited in the present application include, but are not limited to patents, patent applications, articles, books, operation manuals and papers, are hereby incorporated herein by reference in their entirety.

The pharmaceutical composition of the present invention comprises the compound of formula I or formula II, or the optical isomer thereof, the pharmaceutically acceptable salt thereof or a prodrug thereof in the first aspect of the present invention.

In the present application, unless otherwise specified, the term "pharmaceutically acceptable salt" refers to a salt which is suitable for tissue contact of a subject without causing undue side effects. The salt in the present application is mainly a pharmaceutically acceptable acid addition salt and a pharmaceutically acceptable alkali addition salt.

The term "pharmaceutically acceptable acid addition salt" refers to a salt formed with inorganic or organic acids while maintaining the biological effectiveness of a free alkali without other side effects. The inorganic acid salt comprises hydrochloride, hydrobromide, sulfate, nitrate, phosphate and the like; and the organic acid salt comprises formates, acetates, 2,2-dichloroacetates, trifluoroacetates, propionates, caproates, caprylates, caprates, undecenates, glycolates, gluconates, lactates, sebacates, adipates, glutarates, malonates, oxalates, maleates, succinates, fumarates, tartrates, citrates, palmitates, stearates, oleates, cinnamates, laurates, malates, glutamates, pyroglutamates, aspartates, benzoates, methanesulfonates, benzenesulfonates, p-toluenesulfonates, alginates, ascorbates, salicylates, 4-aminosalicylates, naphthalene disulfonates and the like. These salts can be prepared by methods known in the art.

The term "pharmaceutically acceptable alkali addition salt" refers to a salt formed with inorganic or organic alkalis while maintaining the biological effectiveness of a free acid without other side effects. The salts derived from the inorganic alkalis include but are not limited to sodium salts, potassium salts, lithium salts, ammonium salts, calcium salts, magnesium salts, ferric salts, zinc salts, copper salts, manganese salts, aluminum salts and the like. The preferred inorganic salts are ammonium salts, sodium salts, potassium salts, calcium salts and magnesium salts. The salts derived from the organic alkalis include but are not limited to salts of primary amines, secondary amines and tertiary amines, and substituted amines including natural substituted amines, cyclic amines, and basic ion exchange resins, such as ammonia, isopropylamine, trimethylamine, diethylamine, triethylamine, tripropylamine, ethanolamine, diethanolamine, triethanolamine, dimethylethanolamine, 2-dimethylaminoethanol, 2-diethylaminoethanol, dicyclohexylamine, lysine, arginine, histidine, caffeine, procaine, choline, betaine, ethylenediamine, glucosamine, methylglucamine, theobromine, purine, piperazine, piperidine, N-ethylpiperidine, polyamine resins and the like. The preferred organic alkalis comprise diethylamine, ethanolamine, trimethylamine, dicyclohexylamine, choline and caffeine. These salts can be prepared by methods known in the art.

The present invention further comprises the prodrug of the compound. In the present application, the term "prodrug" indicates a compound that can be converted under physiological conditions or by solvolysis to the biologically active compound of the present invention. Therefore, the term "prodrug" refers to a pharmaceutically acceptable metabolic precursor of the compound of the present invention. The prodrug may not be active when administered to a subject in need thereof, but is converted *in vivo* to the active compound of the present invention. The prodrug is generally rapidly converted in vivo to generate a parent compound of the present invention, for example, by hydrolysis in blood. The prodrug compound generally has the advantages of solubility, histocompatibility or sustained release in mammalian organisms. A specific method for preparing the prodrug can refer to Saulnier, M.G., et al., Bioorg. Med. Chem. Lett. 1994, 4, 1985-1990; and Greenwald, R.B., et al., J.Med.Chem. 2000, 43, 475.

The term "pharmaceutically acceptable" used herein refers to a substance (e.g., a carrier or a diluent) that does not affect the biological activity or properties of the compound of the present invention. Besides, the substance is relatively non-toxic, i.e., the substance can be administered to an individual without causing an undesirable biological response or interacting in an undesirable manner with any component contained in the composition.

The term "treatment" and other similar synonyms used herein comprise the following meanings:

(i) preventing the occurrence of a disease or condition in a mammal, particularly when such mammal is susceptible to the disease or condition but has not yet been diagnosed as having the disease or condition;
(ii) inhibiting the disease or condition, i.e., arresting its development;
(iii) alleviating the disease or condition, i.e., causing the state of the disease or condition to resolve; or
(iv) alleviating symptoms caused by the disease or condition.

**Brief Description of the Drawings**

FIG. 1 shows the effect of a compound K18 on the body weight of ICR mice, wherein (A) shows a single administration experiment; and (B) shows a multiple administration experiment; and

FIG. 2 shows the histopathological changes of the liver, kidneys and heart of mice in a vehicle group and mice treated with multiple administration (50 mg/kg) and single administration (500 mg/kg) of the compound K18, wherein the scale bar: 100 μM.

## DETAILED DESCRIPTION

**Example 1 2-[(2,2-dimethylpropyl){[4-(4-methylpiperazin-1-yl)phenyl]methyl}amino]pyrimidine-4-carbonitrile (K1)**

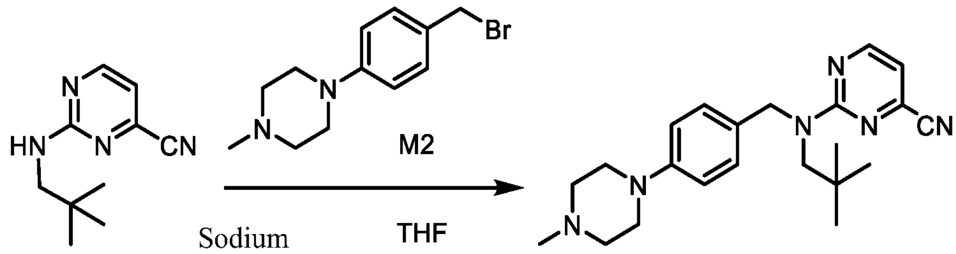


M1 K1

A compound M1 (500 mg, 2.47 mmol) was dissolved in 8 mL of anhydrous tetrahydrofuran, cooled to 0°C, NaH (178 mg, 7.41 mmol) was added portionwise while stirring, and moved to room temperature for reaction for 2 h. A compound M2 (732 mg, 2.72 mmol) was added while stirring and the mixture reacted at room temperature for 2 h. After the reaction was finished, 10 mL of water was added to quench the reaction, tetrahydrofuran was removed by distillation under reduced pressure, water was used for extraction twice, and the organic phases were combined and dried over anhydrous sodium sulfate. The dried organic phase was purified by column chromatography (petroleum ether: ethyl acetate = 20:1) to obtain a white solid, namely a compound K1, with the yield of 60.3%. $^1$H NMR (400 MHz, DMSO-$d_6$) δ 8.60 (d, J = 4.7 Hz, 1H), 7.12 (d, J = 4.7 Hz, 1H), 7.01 (d, J = 8.1 Hz, 2H), 6.84 (d, J = 8.3 Hz, 2H), 4.80 (s, 2H), 3.47 (s, 2H), 3.07 (t, J = 5.0 Hz, 4H), 2.41 (t, J = 4.9 Hz, 4H), 2.20 (s, 3H), 0.94 (s, 9H); ESI-MS m/z: 379.5[M+H]$^+$.

**Example 2 2-[(2,2-dimethylpropyl)[(4-phenylphenyl)methyl]amino]pyrimidine-4-carbonitrile (K2)**

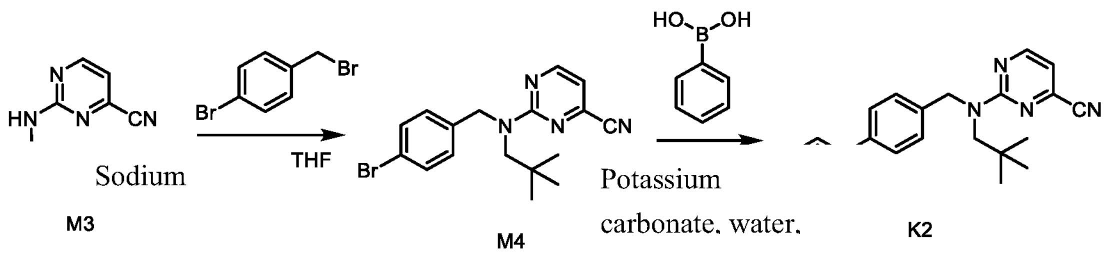


M3 M4 K2

Referring to the synthesis method of example 1, an intermediate M4 was prepared.

The intermediate M4 (291 mg, 0.81 mmol) was dissolved in a mixed solution of 1 mL of water and 9 mL of DMF, phenylborodiol (108 mg, 0.89 mmol) and potassium carbonate (223 mg, 1.616 mmol) were added successively while stirring, and the mixture was vacuumized and heated to 80°C for reaction for 4 h under argon protection. After the reaction was finished, the reaction solution was returned to room temperature, added with 20 mL of water for dilution and extracted twice with ethyl acetate. The organic phases were combined, washed twice with a saturated salt solution and dried over anhydrous sodium sulfate. The dried organic phase was purified by column chromatography (petroleum ether: ethyl

acetate = 12:1) to obtain a white solid, namely a compound K2, with the yield of 54.7%. $^1$H NMR (400 MHz, DMSO-$d_6$) δ 8.65 (s, 1H), 7.69-7.56 (m, 4H), 7.45 (q, J = 7.2 Hz, 2H), 7.36 (t, J = 6.7 Hz, 1H), 7.30-7.15 (m, 3H), 4.96 (s, 2H), 3.58 (s, 2H), 0.99 (d, J = 13.6 Hz, 9H); ESI-MS m/z: 357.4[M+H]$^+$.

**Example 3 2-[(2,2-dimethylpropyl) ({4-[4-(methylthio)phenyl]phenyl}methyl)amino]pyrimidine-4-carbonitrile (K3)**

Referring to the synthesis method of example 2, the raw material phenylborodiol was replaced by 4-methylthiophenylboron diol to obtain a white solid, namely a K3 compound, with the yield of 48.6%. $^1$H NMR (400 MHz, DMSO-$d_6$) δ 8.63 (s, 1H), 7.63-7.55 (m, 4H), 7.35-7.30 (m, 2H), 7.23 (d, J = 7.9 Hz, 2H), 7.17 (d, J = 4.7 Hz, 1H), 4.95 (s, 2H), 3.58 (s, 2H), 2.51 (d, J = 1.9 Hz, 3H), 0.97 (s, 9H); ESI-MS m/z: 403.4[M+H]$^+$.

**Example 4 2-[(2,2-dimethylpropyl){[4-(4-fluorophenyl)phenyl] methyl}amino] pyrimidine-4-carbonitrile (K4)**

Referring to the synthesis method of example 2, the raw material phenylborodiol was replaced by 4-fluorophenyl boranediol to obtain a white solid, namely a K4 compound, with the yield of 47.1%. $^1$H NMR (400 MHz, DMSO-$d_6$) δ 8.63 (s, 1H), 7.62 (d, J = 20.0 Hz, 4H), 7.49 (s, 2H), 7.24 (s, 2H), 7.17 (s, 1H), 4.95 (s, 2H), 3.58 (s, 2H), 0.97 (s, 9H); ESI-MS m/z: 375.4[M+H]$^+$.

**Example 5 2-[(2,2-dimethylpropyl){[4-(4-methoxyphenyl)phenyl] methyl} amino] pyrimidine-4-carbonitrile (K5)**

Referring to the synthesis method of example 2, the raw material phenylborodiol was replaced by 4-methoxyphenyl borondiol to obtain a white solid, namely a K5 compound, with the yield of 44.6%. $^1$H NMR (400 MHz, DMSO-$d_6$) δ 8.63 (s, 1H), 7.55 (t, J = 8.7 Hz, 4H), 7.20 (d, J = 7.8 Hz, 2H), 7.16 (d, J = 4.7 Hz, 1H), 7.00 (d, J = 8.3 Hz, 2H), 4.93 (s, 2H), 3.78 (s, 3H), 3.56 (s, 2H), 0.97 (s, 9H); ESI-MS m/z: 387.4[M+H]$^+$.

**Example 6 2-[(2,2-dimethylpropyl) ({4-[4-(methylsulfonyl)phenyl]phenyl}methyl)amino]pyrimidine-4-carbonitrile (K6)**

Referring to the synthesis method of example 2, the raw material phenylborodiol was replaced by 4-methanesulfonyl phenylborodiol to obtain a white solid, namely a K6 compound, with the yield of 53.7%. $^1$H NMR (400 MHz, DMSO-$d_6$) δ 8.62 (s, 1H), 7.98 (d, J = 8.5 Hz, 2H), 7.90 (d, J = 8.2 Hz, 2H), 7.69 (d, J = 7.9 Hz, 2H), 7.29 (d, J = 7.8 Hz, 2H), 7.17 (d, J = 4.7 Hz, 1H), 4.97 (s, 2H), 3.59 (s, 2H), 3.24 (s, 3H), 0.97 (s, 9H); ESI-MS m/z: 435.4[M+H]$^+$.

**Example 7 2-({[4-(5-cyanothiophen-2-yl)phenyl]methyl}(2,2-dimethylpropyl)amino)pyrimidine-4-carbonitrile (K7)**

Referring to the synthesis method of example 2, the raw material phenylborodiol was replaced by 5-(dihydroxyboryl)thiophene-2-carbonitrile to obtain a white solid, namely a K7 compound, with the yield of 31.7%. $^1$H NMR (400 MHz, DMSO-$d_6$) δ 8.62 (s, 1H), 7.97 (d, J = 4.0 Hz, 1H), 7.69 (d, J = 7.9 Hz, 2H), 7.64 (d, J = 4.0 Hz, 1H), 7.25 (d, J = 7.9 Hz, 2H), 7.17 (d, J = 4.7 Hz, 1H), 4.93 (s, 2H), 3.58 (s, 2H), 0.96 (s, 9H); ESI-MS m/z: 388.4[M+H]$^+$.

**Example 8 2-({[4-(6-chloropyridin-3-yl)phenyl]methyl}(2,2-dimethylpropyl)amino)pyrimidine-4-carbonitrile (K8)**

Referring to the synthesis method of example 2, the raw material phenylborodiol was replaced by (2-chloropyridin-5-yl)boranediol to obtain a white solid, namely a K8 compound, with the yield of 34.5%. $^1$H NMR (400 MHz, DMSO-$d_6$) δ 8.71 (s, 1H), 8.63 (s, 1H), 8.13 (d, J = 8.8 Hz, 1H), 7.68 (d, J = 7.9 Hz, 2H), 7.59 (d, J = 8.4 Hz, 1H), 7.28 (d, J = 7.8 Hz, 2H), 7.18 (d, J = 4.7 Hz, 1H), 4.96 (s, 2H), 3.59 (s, 2H), 0.97 (s, 9H); ESI-MS m/z: 392.4[M+H]$^+$.

**Example 9 2-({[4-(3,4-dichlorophenyl)phenyl]methyl}(2,2-dimethylpropyl)amino)pyrimidine-4-carbonitrile (K9)**

Referring to the synthesis method of example 2, the raw material phenylborodiol was replaced by (1,2-dichlorophen-4-yl)borodiol to obtain a white solid, namely a K9 compound, with the yield of 52.9%. $^1$H NMR (400 MHz, DMSO-$d_6$) δ 8.62 (s, 1H), 7.89 (d, J = 2.1 Hz, 1H), 7.67 (s, 1H), 7.65-7.61 (m, 3H), 7.24 (d, J = 8.0 Hz, 2H), 7.16 (d, J = 4.7 Hz, 1H), 4.94 (s, 2H), 3.58 (s, 2H), 0.96 (s, 9H); ESI-MS m/z: 425.4[M+H]$^+$.

**Example 10 2-[(2,2-dimethylpropyl){[4-(5-methylthiophen-2-yl)phenyl]methyl}amino]pyrimidine-4-carbonitrile (K10)**

Referring to the synthesis method of example 2, the raw material phenylborodiol was replaced by (5-methylthiophen-2-yl)borodiol to obtain a white solid, namely a K10 compound, with the yield of 34.8%. $^1$H NMR (400 MHz, DMSO-$d_6$) δ 8.62 (s, 1H), 7.49 (d, J = 8.0 Hz, 2H), 7.23 (d, J = 3.6 Hz, 1H), 7.19-7.12 (m, 3H), 6.79 (dd, J = 3.6, 1.2 Hz, 1H), 4.89 (s, 2H), 3.56 (s, 2H), 2.44 (d, J = 1.1 Hz, 3H), 0.95 (s, 9H); ESI-MS m/z: 377.4[M+H]$^+$.

**Example 11 4-(4-{[(4-cyanopyrimidin-2-yl) (2,2-dimethylpropyl)amino]methyl}phenyl)methyl benzoate (K11)**

a) Referring to the synthesis method of example 2, the raw material phenylborodiol was replaced by 4-(dihydroxyboryl)methyl benzoate to obtain a white solid, namely a K11 compound, with the yield of 44.7%. $^1$H NMR (400 MHz, DMSO-$d_6$) δ 8.63 (s, 1H), 8.01 (d, J = 8.4 Hz, 2H), 7.79 (d, J = 8.4 Hz, 2H), 7.68 (d, J = 7.9 Hz, 2H), 7.27 (d, J = 7.9 Hz, 2H), 7.17 (d, J = 4.7 Hz, 1H), 4.96 (s, 2H), 3.87 (s, 3H), 3.59 (s, 2H), 0.97 (s, 9H); ESI-MS m/z: 415.4[M+H]$^+$.

**Example 12 2-({[4-(4-chloro-3-fluorophenyl)phenyl]methyl}(2,2-dimethylpropyl)amino)pyrimidine-4-carbonitrile (K12)**

Referring to the synthesis method of example 2, the raw material phenylborodiol was replaced by (1-chloro-2-fluorobenzen-4-yl)borodiol to obtain a white solid, namely a K12 compound, with the yield of 51.8%. $^1$H NMR (400 MHz, DMSO-$d_6$) δ 8.62 (s, 1H), 7.71 (dd, J = 11.0, 2.1 Hz, 1H), 7.64 (t, J = 8.2 Hz, 3H), 7.52 (dd, J = 8.4, 2.1 Hz, 1H), 7.25 (d, J = 7.9 Hz, 2H), 7.16 (d, J = 4.7 Hz, 1H), 4.95 (s, 2H), 3.58 (s, 2H), 0.96 (s, 9H); ESI-MS m/z: 409.4[M+H]$^+$.

**Example 13 2-[(2,2-dimethylpropyl) ({4-[4-(4-methylpiperazin-1-yl)phenyl]phenyl}methyl)amino]pyrimidine-4-carbonitrile (K13)**

Referring to the synthesis method of example 2, the raw material phenylborodiol was replaced by 4-(4-methyl-1-piperazin)phenylborodiol to obtain a white solid, namely a K13 compound, with the yield of 68.7%. $^1$H NMR (400 MHz, $CDCl_3$) δ 8.42 (d, J = 4.7 Hz, 1H), 7.47 (d, J = 8.1 Hz, 4H), 7.17 (s, 2H), 6.98 (d, J = 8.8 Hz, 2H), 6.74 (d, J = 4.7 Hz, 1H), 4.99 (s, 2H), 3.58 (s, 2H), 3.29-3.23 (m, 4H), 2.59 (t, J = 5.0 Hz, 4H), 2.36 (s, 3H), 1.01 (s, 9H); ESI-MS m/z: 455.6[M+H]$^+$.

**Example 14 2-[(cyclohexylmethyl)[(4-phenylphenyl)methyl]amino]pyrimidine-4-carbonitrile (K14)**

Referring to the synthesis method of example 2, a white solid, namely a K14 compound, with the yield of 53.1% was obtained. $^1$H NMR (400 MHz, DMSO-$d_6$) δ 8.65 (s, 1H), 7.61 (m, 4H), 7.44 (m, 2H), 7.32 (m, 3H), 7.15 (m, 1H), 4.88 (s, 2H), 3.46 (m, 2H), 1.81 (s, 1H), 1.62 (m, 5H), 1.14 (m, 3H), 0.97 (m, 2H); ESI-MS m/z: 383.5[M+H]$^+$.

**Example 15 2-[(cyclohexylmethyl) ({4-[4-(4-methylpiperazin-1-yl)phenyl]phenyl}methyl)amino]pyrimidine-4-carbonitrile (K15)**

Referring to the synthesis method of example 2, a white solid, namely a K15 compound, with the yield of 60.7% was obtained. $^1$H NMR (400 MHz, DMSO-$d_6$) δ 8.63 (s, 1H), 7.51 (dd, J = 15.1, 8.1 Hz, 4H), 7.23 (d, J = 7.7 Hz, 2H), 7.14 (d, J = 4.7 Hz, 1H), 6.99 (d, J = 8.4 Hz, 2H), 4.85 (s, 2H), 3.16 (s, 4H), 2.45 (s, 4H), 2.22 (s, 3H), 1.81 (s, 1H), 1.62 (d, J = 16.2 Hz, 5H), 1.15 (s, 3H), 0.97 (d, J = 11.8 Hz, 2H); ESI-MS m/z: 481.7[M+H]$^+$.

**Example 16 2-[(cyclohexylmethyl) ({4-[4-(methylsulfonyl)phenyl]phenyl}methyl)amino]pyrimidine-4-carbonitrile (K16)**

Referring to the synthesis method of example 2, a white solid, namely a K16 compound, with the yield of 49.3% was obtained. $^1$H NMR (400 MHz, DMSO-$d_6$) δ 8.64 (s, 1H), 7.98 (d, J = 8.5 Hz, 2H), 7.91 (d, J = 8.3 Hz, 2H), 7.70 (d, J = 7.8 Hz, 2H), 7.35 (d, J = 7.9 Hz, 2H), 7.15 (d, J = 4.7 Hz, 1H), 4.90 (s, 2H), 3.48 (d, J = 7.4 Hz, 2H), 3.24 (s, 3H), 1.86-1.76 (m, 1H), 1.62 (d, J = 15.1 Hz, 5H), 1.14 (d, J = 8.0 Hz, 3H), 0.98 (d, J = 11.8 Hz, 2H); ESI-MS m/z: 461.6[M+H]$^+$.

**Example 17 2-(neopentyl(4-(thiazol-4-yl)benzyl)amino)pyrimidine-4-carbonitrile (K17)**

Referring to the synthesis method of example 2, a white solid, namely a K17 compound, with the yield of 32.9% was obtained. $^1$H NMR (400 MHz, DMSO-$d_6$) δ 9.17 (s, 1H), 8.62 (s, 1H), 8.09 (s, 1H), 7.91 (s, 2H), 7.22 (s, 3H), 4.93 (s, 2H), 3.57 (s, 2H), 0.96 (s, 9H); ESI-MS m/z: 364.3[M+H]$^+$.

**Example 18 2-((4-(2-(4-methylpiperazin-1-yl)thiazol-4-yl)benzyl) (neopentyl)amino)pyrimidine-4-carbonitrile (K18)**

Referring to the synthesis method of example 2, a white solid, namely a K18 compound, with the yield of 48.1% was obtained. $^1$H NMR (400 MHz, DMSO-$d_6$) δ 8.61 (s, 1H), 7.75 (t, J = 8.6 Hz, 2H), 7.26-7.07 (m, 4H), 4.91 (d, J = 8.2 Hz, 2H), 3.56 (s, 2H), 3.43 (s, 4H), 2.42 (s, 4H), 2.22 (s, 3H), 0.95 (s, 9H); ESI-MS m/z: 462.6 [M+H]$^+$.

**Example 19** (1R,2R)-2-(8-fluoro-2,3,4,9-tetrahydro-1H-pyrido[3,4-b]indole-2-carbonyl)-N-(4-oxotetrahydrofuran-3-yl) cyclohexane-1-carboxamide (K19)

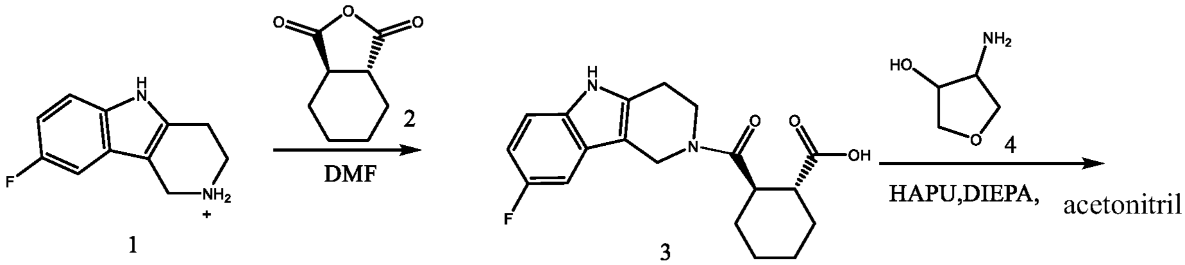


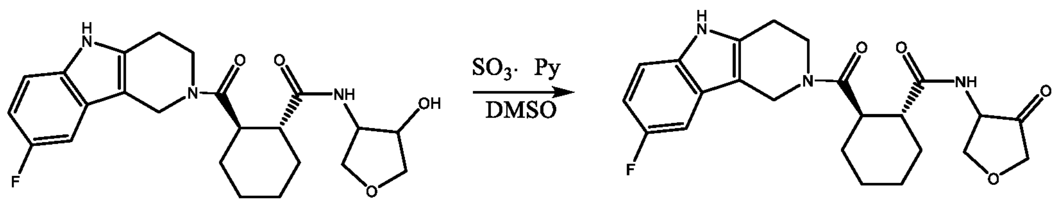


5

K19

A compound 1 (530 mg, 2.34 mmol) and a compound 2 (300 mg, 1.95 mmol) were weighed and put into a 100 mL eggplant-shaped flask, dissolved in 25 mL of DMF, and stirred at room temperature for 12 h. After the reaction was finished, the reaction solution was diluted with DCM, washed with 10% hydrochloric acid, dried over anhydrous sodium sulfate, and separated by column chromatography (dichloromethane: methanol = 50: 1) to obtain a yellowish white solid, namely an intermediate 3.

The intermediate 3 (100 mg, 0.29 mmol) and HATU (121 mg, 0.32 mmol) were weighed and put into a 25 mL eggplant-shaped flask, 10 mL of acetonitrile was added, and DIPEA (191 μL, 1.16 mmol) and a compound 4 (33 mg, 0.32 mmol) were added for reaction for 10 min. After the reaction was finished, the reaction solution was dried and purified by column chromatography (dichloromethane: methanol = 50: 1) to obtain a yellowish white solid, namely an intermediate 5.

The intermediate 5 was placed into a 25 mL two-necked flask under $N_2$ protection, 7 mL of anhydrous DCM and DIPEA (386 μL, 0.92 mmol) was added and dissolved, and the mixture was placed under a low-temperature cold trap and cooled to -15°C; and a sulfur trioxide pyridine complex (146 mg, 0.88 mmol) was additionally weighed and dissolved in 1 mL of anhydrous DMSO to form a mixed solution, and the mixed solution was added into the reaction solution in the two-necked flask at -15°C for reaction for 1 h at low temperature. After the reaction was finished, the reaction solution was purified by column chromatography (petroleum ether: ethyl acetate: methanol = 16:4:1) to obtain a white solid, namely a compound K19, with the yield of 68%. M.P.146-148°C, $^1$H NMR (400 MHz, DMSO-$d_6$) δ 10.98 (s, 1H), 7.75 (dd, $J$ = 16.0, 8.3 Hz, 1H), 7.39-7.09 (m, 2H), 6.85 (dtd, $J$ = 9.1, 6.1, 2.9 Hz, 1H), 4.84-4.32 (m, 2H), 4.14-3.73 (m, 3H), 3.12-2.89 (m, 2H), 2.86-2.63 (m, 2H), 2.18 (dt, $J$ = 17.4, 7.6 Hz, 1H), 1.97-1.54 (m, 7H), 1.53-1.07 (m, 6H); $^{13}$C NMR (101 MHz, DMSO) δ 215.55, 175.04, 173.87, 170.82, 158.33, 156.03, 135.42, 132.87, 126.01, 112.14, 108.84, 106.89, 103.17, 102.58, 60.23, 56.09, 49.07, 46.42, 43.18, 42.55, 41.83, 41.43, 39.37, 35.69, 30.44, 29.57, 29.16, 25.70, 24.61, 23.49, 21.22, 18.18, 14.55; MS (ESI): 426.4 [M+H]$^+$.

**Example 20** (1R,2R)-2-(2,3,4,5-tetrahydro-1H-pyrido[4,3-b]indole-2-carbonyl)-N-(4-oxotetrahydrofuran-3-yl)-cyclohexane-1-carboxamide (K20)

The preparation method of the present example referred to the preparation steps of example 19. The compound 1 was replaced by 2,3,4,5-tetrahydro-1H-pyrido[4,3-b]indole-2-ium to finally obtain a white solid, namely a compound K20, with the yield of 71%. M.P. 137-140°C; $^1H$ NMR (400 MHz, DMSO-$d_6$) δ 10.88 (s, 1H), 8.30-8.01 (m, 1H), 7.48 (d, *J* = 7.6 Hz, 0H), 7.38 (d, *J* = 7.7 Hz, 1H), 7.32-7.24 (m, 1H), 7.08-6.91 (m, 2H), 4.78-4.46 (m, 2H), 4.26-3.52 (m, 7H), 3.09-2.81 (m, 2H), 2.74-2.54 (m, 2H), 1.86-1.61 (m, 4H), 1.40-1.14 (m, 4H); $^{13}C$ NMR (101 MHz, CDCl3) δ 211.38, 211.31, 175.85, 175.79, 174.54, 174.16, 136.00, 131.28, 125.61, 121.72, 119.67, 117.50, 110.85, 106.62, 70.07, 69.90, 69.71, 69.49, 54.76, 54.69, 46.33, 46.17, 43.39, 42.96, 40.07, 39.87, 29.27, 28.86, 25.27, 24.49, 23.29; MS (ESI) :410.4 $[M+H]^+$.

**Example 21** (1R,2R)-2-(8-fluoro-2,3,4,9-tetrahydro-1H-pyrido[3,4-b]indole-2-carbonyl)-N-((S)-4-oxotetrahydrofuran-3-yl)cyclohexane-1-carboxamide (K21)

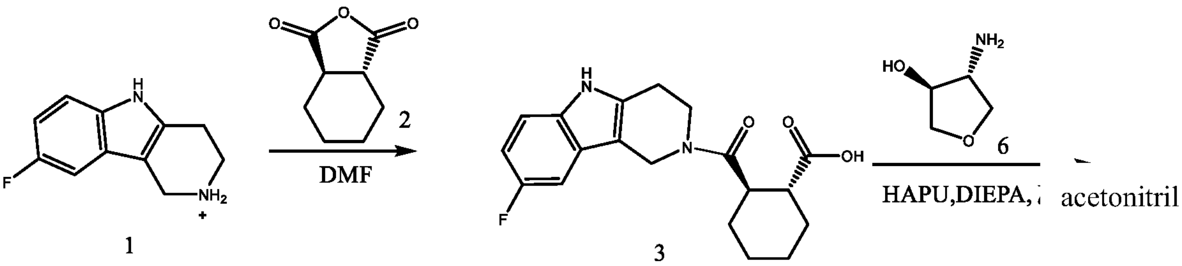


1

3

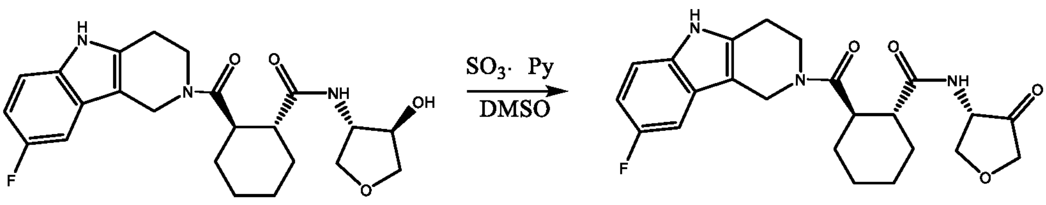


7

K21

The preparation method of the present example referred to the preparation steps of example 19. The compound 4 was replaced by a compound 6 to obtain a white solid product, namely a compound K21, with the yield of 67%. $^1H$ NMR (400 MHz, DMSO) δ 11.01 (s, 1H), 8.15-8.02 (m, 1H), 7.32-7.14 (m, 2H), 6.86 (tdd, J = 9.2, 4.8, 2.6 Hz, 1H), 4.74-4.42 (m, 2H), 4.34-3.38 (m, 7H), 3.05-2.53 (m, 4H), 1.92-1.57 (m, 4H), 1.36-1.23 (m, 4H).

**Example 22** (1R,2R)-2-(6,8-difluoro-2,3,4,9-tetrahydro-1H-pyrido[3,4-b]indole-2-carbonyl)-N-((S)-4-oxotetrahydrofuran-3-yl)cyclohexane-1-carboxamide (K22)

The preparation method of the present example referred to the preparation steps of example 19 to obtain a white solid product, namely a compound K22, with the yield of 66%. $^1H$ NMR (400 MHz, DMSO) δ 11.48 (s, 1H), 8.30-8.02 (m, 1H), 7.21-7.05 (m, 1H), 6.88 (d, J = 12.6 Hz, 1H), 4.70 (q, J = 13.9 Hz, 2H), 4.51-3.36 (m, 7H), 3.06-2.86 (m, 2H), 2.74-2.57 (m, 2H), 1.71-1.27 (m, 8H).

**Example 23** (1R,2R)-2-(8-chloro-2,3,4,5-tetrahydro-1H-pyrido[4,3-b]indole-2-carbonyl)-N-((S)-4-oxotetrahydrofuran-3-yl)cyclohexane-1-carboxamide (K23)

The preparation method of the present example referred to the preparation steps of example 19 to obtain a white solid product, namely a compound K23, with the yield of 65%. $^1H$ NMR (400 MHz, DMSO) δ 11.12 (d, J = 4.3 Hz, 1H), 8.30-8.05 (m, 1H), 7.63-7.42 (m, 1H), 7.30 (dd, J = 8.6, 1.9 Hz, 1H), 7.03 (ddt, J = 7.9, 3.6, 1.9 Hz, 1H), 4.78-4.49 (m, 2H), 4.48-3.39 (m, 7H), 3.07-2.82 (m, 2H), 2.79-2.57 (m, 2H), 1.86-1.11 (m, 8H).

**Example 24** (1R,2R)-2-(8-bromo-2,3,4,5-tetrahydro-1H-pyrido[4,3-b]indole-2-carbonyl)-N-((S)-4-oxotetrahydrofuran-3-yl)cyclohexane-1-carboxamide (K24)

The preparation method of the present example referred to the preparation steps of example 19 to obtain a yellowish white solid, namely a compound K24, with the yield of 69%. $^1$H NMR (400 MHz, DMSO) δ 11.14 (d, J = 5.1 Hz, 1H), 8.38-7.94 (m, 1H), 7.80-7.51 (m, 1H), 7.38-7.05 (m, 3H), 4.88-4.43 (m, 2H), 4.35-3.45 (m, 7H), 3.20-2.86 (m, 2H), 2.87-2.58 (m, 2H), 1.83-1.66 (m, 4H), 1.36-1.19 (m, 4H).

**Example 25** (1R,2R)-2-(6-(trifluoromethoxy)-2,3,4,5-tetrahydro-1H-pyrido[4,3-b]indole-2-carbonyl)-N-(4-oxotetrahydrofuran-3-yl)-cyclohexane-1-carboxamide (K25)

The preparation method of the present example referred to the preparation steps of example 19 to obtain a white solid product, namely a compound K25, with the yield of 40%. $^1$H NMR (300 MHz, DMSO) δ 11.49 (s, 1H), 8.32-8.05 (m, 1H), 7.57-7.39 (m, 1H), 7.04 (d, J = 5.4 Hz, 2H), 4.83-4.55 (m, 2H), 4.40-3.44 (m, 7H), 3.06-2.82 (m, 2H), 2.78-2.54 (m, 2H), 1.81 (d, J = 15.7 Hz, 4H), 1.26 (d, J = 12.3 Hz, 4H).

**Example 26** (1R,2R)-2-(6-(trifluoromethoxy)-2,3,4,5-tetrahydro-1H-pyrido[4,3-b]indole-2-carbonyl)-N-(S)-(4-oxotetrahydrofuran-3-yl)-cyclohexane-1-carboxamide (K26)

The preparation method of the present example referred to the preparation steps of example 19 to obtain a white solid, namely a compound K26, with the yield of 42%. $^1$H NMR (300 MHz, DMSO) δ 11.49 (s, 1H), 8.29-8.03 (m, 1H), 7.56-7.39 (m, 1H), 7.04 (d, J = 5.3 Hz, 2H), 4.82-4.64 (m, 2H), 4.57-3.39 (m, 7H), 3.07-2.89 (m, 2H), 2.78-2.54 (m, 2H), 1.72 (s, 4H), 1.33-1.23 (m, 4H).

**Example 27** (1R,2R)-2-(6-fluoro-2,3,4,5-tetrahydro-1H-pyrido[4,3-b]indole-2-carbonyl)-N-(S)-(4-oxotetrahydrofuran-3-yl)-cyclohexane-1-carboxamide (K27)

The preparation method of the present example referred to the preparation steps of example 19 to obtain a white solid, namely a compound K27, with the yield of 66%. $^1$H NMR (400 MHz, DMSO) δ 11.48 (s, 1H), 8.30-8.05 (m, 1H), 7.56-7.41 (m, 1H), 7.04 (d, J = 6.5 Hz, 2H), 4.80-4.54 (m, 2H), 4.54-3.38 (m, 7H), 3.06-2.53 (m, 4H), 1.91-1.25 (m, 8H).

**Example 28** (1R,2R)-2-(6-fluoro-2,3,4,5-tetrahydro-1H-pyrido[4,3-b]indole-2-carbonyl)-N-(S)-(4-oxotetrahydrofuran-3-yl)-cyclohexane-1-carboxamide (K28)

The preparation method of the present example referred to the preparation steps of example 19 to obtain a white solid, namely a compound K28, with the yield of 51%. $^1$H NMR (400 MHz, DMSO) δ 11.37 (s, 1H), 8.34-8.04 (m, 1H), 7.38-7.20 (m, 1H), 6.96-6.88 (m, 2H), 4.78-4.52 (m, 2H), 4.51-3.44 (m, 7H), 3.08-2.90 (m, 2H), 2.81-2.57 (m, 2H), 1.73 (m, 4H), 1.25 (m, 4H).

**Example 29** (1R,2R)-2-(6-methoxy-2,3,4,5-tetrahydro-1H-pyrido[4,3-b]indole-2-carbonyl)-N-(S)-(4-oxotetrahydrofuran-3-yl)-cyclohexane-1-carboxamide (K29)

The preparation method of the present example referred to the preparation steps of example 19 to obtain a white solid, namely a compound K29, with the yield of 63%. $^1$H NMR (400 MHz, DMSO) δ 10.96 (d, J = 4.1 Hz, 1H), 8.31-7.99 (m, 1H), 7.12-6.83 (m, 2H), 6.63 (dd, J = 7.6, 5.7 Hz, 1H), 4.77-4.40 (m, 2H), 4.37-3.92 (m, 3H), 3.89 (s, 3H), 3.87-3.42 (m, 4H), 3.04-2.54 (m, 5H), 1.91-1.61 (m, 5H), 1.35-1.13 (m, 4H).

**Example 30** (1R,2R)-2-(6-methoxy-2,3,4,5-tetrahydro-1H-pyrido[4,3-b]indole-2-carbonyl)-N-(4-oxotetrahydrofuran-3-yl)-cyclohexane-1-carboxamide (K30)

The preparation method of the present example referred to the preparation steps of example 19 to obtain a white solid, namely a compound K30, with the yield of 43%. $^1$H NMR (300 MHz, DMSO) δ 10.96 (s, 1H), 8.33-8.02 (m, 1H), 7.15-6.83 (m, 2H), 6.63 (dd, J = 7.7, 4.2 Hz, 1H), 4.74-4.50 (m, 2H), 4.49-3.88 (m, 7H), 3.87-3.38 (m, 3H), 3.13-2.76 (m, 2H), 2.75-2.54 (m, 2H), 1.91-1.72 (m, 4H), 1.30 (m, 4H).

**Example 31** (1R,2R)-2-(8-fluoro-6-methoxy-2,3,4,5-tetrahydro-1H-pyrido[4,3-b]indole-2-carbonyl)-N-(S)-(4-oxotetrahydrofuran-3-yl)-cyclohexane-1-carboxamide (K31)

The preparation method of the present example referred to the preparation steps of example 19 to obtain a white solid product, namely a compound K31, with the yield of 68%. $^1$H NMR (400 MHz, DMSO) δ 11.07 (d, J = 3.9 Hz, 1H), 8.30-8.02 (m, 1H), 6.94-6.72 (m, 1H), 6.56 (ddt, J = 11.5, 3.9, 2.0 Hz, 1H), 4.78-4.46 (m, 2H), 4.45-3.88 (m, 7H), 3.87-3.46 (s, 3H), 3.16-2.52 (m, 4H), 2.05-1.45 (m, 4H), 1.44-1.09 (m, 4H).

**Example 32** (1R,2R)-2-(8-fluoro-6-methoxy-2,3,4,5-tetrahydro-1H-pyrido[4,3-b]indole-2-carbonyl)-N-(4-oxotetrahydrofuran-3-yl)-cyclohexane-1-carboxamide (K32)

The preparation method of the present example referred to the preparation steps of example 19 to obtain a white solid, namely a compound K32, with the yield of 58%. $^1$H NMR (400 MHz, DMSO) δ 11.07 (s, 1H), 8.30-8.03 (m, 1H), 6.91-6.75 (m, 1H), 6.60-6.52 (m, 1H), 4.71-4.45 (m, 2H), 4.45-3.88 (m, 7H), 3.87-3.44 (s, 3H), 3.04-2.75 (m, 2H), 2.70-2.54 (m, 2H), 1.82-1.26 (m, 8H).

**Example 33** 2-((4-chlorobenzyl) (neopentyl)amino)pyrimidine-4-carbonitrile (K33)

The preparation method of the present example referred to the preparation steps of example 1 to obtain a white solid product with the yield of 55% by using 4-chlorobenzyl bromide as a raw material. $^1$H NMR (400 MHz, DMSO-$d_6$) δ 8.62 (s, 1H), 7.36 (d, J = 8.4 Hz, 2H), 7.21-7.15 (m, 3H), 4.88 (s, 2H), 3.56 (s, 2H), 0.95 (s, 9H). 13C NMR (101 MHz, CDCl3) δ 162.69, 159.34, 141.29, 136.28, 132.79, 128.68, 128.36, 116.23, 112.24, 57.96, 51.89, 34.75, 28.64. HRMS(ESI) for $C_{17}H_{19}ClN_4$ $[M+H]^+$: calcd, 315.1371; found, 315.1370.

**Example 34** 2-((4-bromobenzyl) (neopentyl)amino)pyrimidine-4-carbonitrile (K34)

The preparation method of the present example referred to the preparation steps of example 1 to obtain a white solid product with the yield of 55% by using 4-bromobenzyl bromide as a raw material. $^1$H NMR (400 MHz, DMSO-$d_6$) δ 8.61 (s, 1H), 7.48 (d, J = 8.2 Hz, 2H), 7.17 (d, J = 4.7 Hz, 1H), 7.11 (d, J = 8.0 Hz, 2H), 4.85 (s, 2H), 3.55 (s, 2H), 0.94 (s, 9H). $^{13}$C NMR (75 MHz, CDCl3) δ 162.68, 159.35, 141.29, 136.82, 131.63, 128.84, 120.85, 116.23, 112.26, 57.98, 51.96, 34.75, 28.63. HRMS(ESI) for $C_{17}H_{19}BrN_4$ $[M+H]^+$: calcd, 359.0866; found, 359.0865.

**Example 35** 2-((naphthalen-2-ylmethyl) (neopentyl)amino)pyrimidine-4-carbonitrile (K35)

The preparation method of the present example referred to the preparation steps of example 1 to obtain a white solid product with the yield of 50%. $^1$H NMR (400 MHz, DMSO-$d_6$) δ 8.60 (s, 1H), 7.89-7.80 (m, 3H), 7.62 (s, 1H), 7.51-7.44 (m, 2H), 7.34 (d, J = 8.5 Hz, 1H), 7.17 (d, J = 4.7 Hz, 1H), 5.08 (s, 2H), 3.63 (s, 2H), 0.98 (s, 9H). $^{13}$C NMR (101 MHz, $CDCl_3$) δ 162.90, 159.35, 141.32, 135.21, 133.33, 132.64, 128.40, 127.70, 126.19, 125.72, 125.29, 116.33, 112.11, 57.91, 52.50, 34.81, 28.71. HRMS(ESI) for $C_{21}H_{22}N_4$ $[M+H]^+$ : calcd, 331.1917; found, 331.1919.

**Example 36** 2-(neopentyl(quinolin-2-ylmethyl)amino)pyrimidine-4-carbonitrile (K36)

The preparation method of the present example referred to the preparation steps of example 1 to obtain a white solid product with the yield of 27%. $^1$H NMR (300 MHz, DMSO-$d_6$) δ 8.75-8.44 (m, 1H), 8.28 (d, J = 8.6 Hz, 1H), 7.94-7.86 (m, 2H), 7.77-7.66 (m, 1H), 7.60-7.50 (m, 1H), 7.31 (d, J = 8.6 Hz, 1H), 7.15 (d, J = 4.7 Hz, 1H), 5.12 (s, 2H), 3.71 (s, 2H), 0.99 (s, 9H). $^{13}$C NMR (101 MHz, $CDCl_3$) δ 162.93, 159.29, 158.69, 147.93, 141.24, 136.59, 129.57, 129.02, 127.59, 127.21, 126.15, 118.62, 116.28, 112.29, 59.41, 55.82, 34.71, 28.58. HRMS(ESI) for $C_{20}H_{21}N_5$ $[M+H]^+$ : calcd, 332.1870; found, 332.1880.

**Example 37** 2-(((4'-(4-(2-hydroxyethyl)piperazin-1-yl)-[1,1'-biphenyl]-4-yl)methyl) (neopentyl)amino)pyrimidine-4-carbonitrile (K37)

The preparation method of the present example referred to the preparation steps of example 1 to obtain a white solid product with the yield of 53%. $^1$H NMR (400 MHz, CDCl3) *δ* 8.45 (d, *J* = 4.7 Hz, 1H), 7.56-7.45 (m, 4H), 7.20 (s, 2H), 7.00 (d, *J* = 8.8 Hz, 2H), 6.77 (d, *J* = 4.7 Hz, 1H), 5.02 (s, 2H), 3.75 (t, *J* = 5.3 Hz, 2H), 3.60 (s, 2H), 3.38-3.27 (m, 4H), 3.13 (q, *J* = 7.4 Hz, 2H), 2.86-2.77 (m, 4H), 2.72 (t, *J* = 5.3 Hz, 2H), 1.03 (s, 9H). $^{13}$C NMR (101 MHz, CDCl3) *δ* 162.87, 159.34, 150.14, 141.26, 139.61, 135.89, 132.25, 127.67, 127.30, 126.64, 116.33, 116.29, 112.03, 77.39, 59.58, 57.57, 52.90, 52.03, 48.64, 45.91, 34.76, 28.68. HRMS(ESI) for $C_{29}H_{36}N_6O$ $[M+H]^+$: calcd, 485.3023; found, 485.3013.

**Example 38** 2-(((4'-(4-hydroxypiperidin-1-yl)-[1,1'-biphenyl]-4-yl)methyl) (neopentyl)amino)pyrimidine-4-carbonitrile (K38)

The preparation method of the present example referred to the preparation steps of example 1 to obtain a white solid product with the yield of 46%. $^1$H NMR (300 MHz, DMSO-*d6)* $\delta$ 8.63 (s, 1H), 7.56-7.43 (m, 4H), 7.23-7.13 (m, 3H), 7.03-6.93 (m, 2H), 4.93 (s, 2H), 4.71 (d, *J* = 4.2 Hz, 1H), 3.69-3.50 (m, 5H), 2.95-2.81 (m, 2H), 1.87-1.74 (m, 2H), 1.54-1.39 (m, 2H), 0.97 (s, 9H). 13C NMR (101 MHz, $CDCl_3$) $\delta$ 162.88, 159.33, 150.43, 141.28, 139.75, 135.74, 131.56, 127.62, 127.29, 126.59, 116.51, 116.34, 112.02, 67.88, 57.70, 52.03, 47.07, 34.77, 34.09, 28.69. HRMS(ESI) for $C_{28}H_{33}N_5O$ $[M+H]^+$: calcd, 456.2758; found, 456.2747.

**Example 39** 2-(((2-methyl-4'-(4-methylpiperazin-1-yl)-[1,1'-biphenyl]-4-yl)methyl) (neopentyl)amino)pyrimidine-4-carbonitrile (K39)

The preparation method of the present example referred to the preparation steps of example 1 to obtain a white solid product with the yield of 54%. $^1$H NMR (300 MHz, $CDCl_3$) $\delta$ 8.46 (d, J = 4.7 Hz, 1H), 7.23 (d, J = 8.7 Hz, 2H), 7.18-7.13 (m, 1H), 6.98 (d, J = 8.7 Hz, 4H), 6.77 (d, J = 4.7 Hz, 1H), 5.00 (s, 2H), 3.61 (s, 2H), 3.35-3.25 (m, 4H), 2.63 (t, J = 5.0 Hz, 4H), 2.40 (s, 3H), 2.27 (s, 3H), 1.03 (s, 9H). $^{13}$C NMR (101 MHz, $CDCl_3$) $\delta$ 162.91, 159.20, 149.88, 141.20, 140.52, 136.01, 135.74, 132.76, 130.05, 129.97, 128.62, 124.12, 116.35, 115.39, 111.97, 57.80, 55.15, 51.90, 48.85, 46.13, 34.78, 28.71, 20.74. HRMS(ESI) for $C_{29}H_{36}N_6$ $[M+H]^+$ : calcd, 469.3074; found, 469.3064.

**Example 40** 2-(((2-chloro-4'-(4-methylpiperazin-1-yl)-[1,1'-biphenyl]-4-yl)methyl) (neopentyl)amino)pyrimidine-4-carbonitrile (K40)

The preparation method of the present example referred to the preparation steps of example 1 to obtain a white solid product with the yield of 51%. $^1$H NMR (300 MHz, $CDCl_3$) $\delta$ 8.46 (s, 1H), 7.36 (d, J = 8.2 Hz, 2H), 7.28-7.19 (m, 2H), 7.12-6.93 (m, 3H), 6.80 (d, J = 4.6 Hz, 1H), 4.98 (s, 2H), 3.61 (s, 2H), 3.31 (s, 4H), 2.62 (s, 4H), 2.39 (s, 3H), 1.03 (s, 9H). HRMS(ESI) for $C_{28}H_{33}ClN_6$ $[M+H]^+$ : calcd, 489.2528; found, 489.2519.

**Example 41** 2-(neopentyl(4-(4-(2-oxopropyl)piperazin-1-yl)benzyl)amino)pyrimidine-4-carbonitrile (K41)

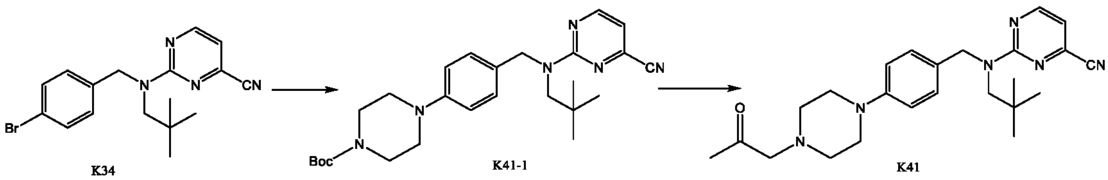

Referring to the preparation steps of example 1, a white solid product K41-1 with the yield of 64% was obtained. $^1$H NMR (400 MHz, DMSO-*d*6) $\delta$ 8.63 (d, *J* = 4.7 Hz, 1H), 7.11 (d, *J* = 4.7 Hz, 1H), 7.04 (d, *J* = 8.1 Hz, 2H), 6.91 (d, *J* = 8.1 Hz, 2H), 4.81 (s, 2H), 3.44 (s, 2H), 3.37 (t, *J* = 4.9 Hz, 4H), 3.14-3.04 (m, 4H), 1.42 (s, 9H), 0.94 (s, 9H).

The intermediate K41-1 (330 mg, 1.41 mg) was added to 10 mL of a cold 4 N hydrochloric acid/dioxane solution and reacted in an ice bath for 30 min. After TLC detected that the reaction was finished, the reaction solution was removed by distillation under reduced pressure, and a small amount of toluene was added until the reaction solution was completely removed. Then the residue was dissolved in 10 mL of DMF, potassium carbonate (487 mg, 3.53 mmol) and chloroacetone (195 mg, 2.11 mmol) were successively added, and the reaction solution was heated to 80°C and reacted under heat preservation for 2 h. After the TLC detected that the reaction was finished, the reaction solution was cooled to room temperature. The reaction solution was added with 20 mL of water and extracted with ethyl acetate (20 mL×3). The organic phases were combined, successively washed with dilute hydrochloric acid (10 mL×2) and a saturated sodium chloride solution (20 mL×2), and dried over anhydrous sodium sulfate for 4 h. The organic phase was subjected to vacuum filtration. A filtrate was concentrated under reduced pressure and purified by column chromatography (petroleum ether: ethyl acetate = 1:1) to obtain 249 mg of a faint yellow solid K41 with the total yield of 42% in two steps. $^1$H NMR (400 MHz, DMSO-d6) $\delta$ 8.61 (d, *J* = 4.7 Hz, 1H), 7.13 (dd, *J* = 4.7, 1.6 Hz, 1H), 7.02 (d, *J* = 8.4 Hz, 2H), 6.85 (d, *J* = 8.3 Hz, 2H), 4.81 (s, 2H), 3.52 (s, 2H), 3.28-3.21 (m, 2H), 3.16-3.03 (m, 4H), 2.59-2.52 (m, 4H), 2.16-2.02 (m, 3H), 0.95 (s, 9H). HRMS(ESI) for $C_{24}H_{32}N_6O$ $[M+H]^+$: calcd, 421.2710; found, 421.2704.

**Example 42** 2-(isobutyl((4'-(4-methylpiperazin-1-yl)-[1,1'-biphenyl]-4-yl)methyl)amino)pyrimidine-4-carbonitrile (K42)

Referring to the preparation steps of example 1, a white solid product with the yield of 46% was obtained. $^1H$ NMR (300 MHz, DMSO-$d_6$) δ 8.66 (s, 1H), 7.59-7.46 (m, 4H), 7.25 (d, J = 7.9 Hz, 2H), 7.17 (d, J = 4.6 Hz, 1H), 7.00 (d, J = 8.6 Hz, 2H), 4.88 (s, 2H), 3.47-3.40 (m, 2H), 3.23-3.12 (m, 4H), 2.49-2.42 (m, 4H), 2.23 (s, 3H), 2.19-2.08 (m, 1H), 0.88 (d, J = 6.6 Hz, 6H). $^{13}C$ NMR (101 MHz, $CDCl_3$) δ 162.18, 159.56, 150.53, 141.56, 139.92, 135.68, 131.81, 127.95, 127.65, 126.64, 116.40, 116.05, 111.87, 55.10, 54.33, 50.61, 48.86, 46.20, 26.78, 20.24. HRMS(ESI) for $C_{27}H_{32}N_6$ $[M+H]^+$ : calcd, 441.2761; found, 441.2753.

**Example 43** N-(4-((4-cyanopyrimidin-2-yl) (neopentyl)amino)methyl)phenyl)-2-(4-methylpiperazin-1-yl)acetamide (K43)

Referring to the preparation steps of example 1, a white solid product with the yield of 43% was obtained. $^1H$ NMR (300 MHz, CDCl3) δ 9.09 (s, 1H), 8.43 (d, *J* = 4.7 Hz, 1H), 7.50 (d, *J* = 8.5 Hz, 2H), 7.22-7.08 (m, 2H), 6.76 (d, *J* = 4.7 Hz, 1H), 4.94 (s, 2H), 3.56 (s, 2H), 3.15 (s, 2H), 2.79-2.41 (m, 8H), 2.36 (s, 3H), 1.01 (s, 9H). $^{13}C$ NMR (101 MHz, CDCl3) δ 168.12, 162.76, 159.26, 141.22, 136.45, 133.68, 127.80, 119.67, 116.29, 112.03, 61.72, 57.71, 54.99, 52.95, 51.98, 45.63, 34.72, 28.64. HRMS(ESI) for $C_{24}H_{33}N_7O$ $[M+H]^+$: calcd, 436.2819; found, 436.2811.

**Example 44** 2-((4-(4-methylpiperazin-1-carbonyl)benzyl) (neopentyl)amino)pyrimidine-4-carbonitrile (K44)

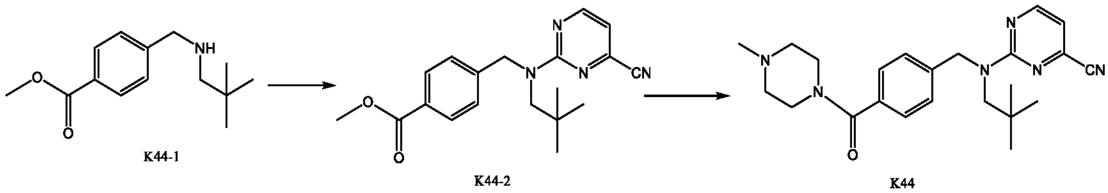

An intermediate K44-1 was dissolved in 10 mL of acetonitrile, DIPEA (2.13 g, 16.48 mmol) was added, and the reaction solution was heated to 80°C and reacted under heat preservation for 16 h. After the TLC detected that the reaction was finished, the reaction solution was cooled to room temperature. The reaction solution was added with 20 mL of water and extracted with ethyl acetate (20 mL×3). The organic phases were combined, successively washed with dilute hydrochloric acid (10 mL×2) and a saturated sodium chloride solution (20 mL×2), and dried over anhydrous sodium sulfate for 4 h. The organic phase was subjected to vacuum filtration. A filtrate was concentrated under reduced pressure and purified by column chromatography (petroleum ether: ethyl acetate = 12:1) to obtain a white solid K44-2 with the yield of 55%. $^1H$ NMR (300 MHz, DMSO-*d*6) δ 8.59 (s, 1H), 7.89 (d, *J* = 8.2 Hz, 2H), 7.28 (d, *J* = 8.0 Hz, 2H), 7.18 (d, *J* = 4.7 Hz, 1H), 4.96 (s, 2H), 3.83 (s, 3H), 3.60 (s, 2H), 0.96 (s, 9H).

The intermediate K44-2 (700 mg, 2.07 mmol) was added to 8 mL of a mixed solution of methanol and water (3:1), then lithium hydroxide (99 mg, 4.14 mmol) was added, and then the mixture reacted in an ice bath for 4 h. After the TLC detected that the reaction was finished, the reaction solution was concentrated, then the pH of the reaction solution was adjusted to about 2 by using a 1 N hydrochloric acid solution in an ice bath to generate a large amount of a white solid, suction filtration was performed, and a filter cake was washed by using ethyl acetate and a small amount of ethanol, and dried overnight in a vacuum environment to obtain a crude carboxylic acid product which was directly used for the next reaction. The obtained carboxylic acid was added to 10 mL of anhydrous tetrahydrofuran, and HATU (787 mg, 2.07 mmol), triethylamine (524 mg, 5.18 mmol) and N-methylpiperazine (249 mg, 2.48 mmol) were successively added and reacted at room temperature for 2 h. After the TLC detected that the reaction was finished, the reaction solution was diluted with 40 mL of ethyl acetate, successively washed with a saturated sodium carbonate solution (15 mL×2), a diluted hydrochloric acid solution (15 mL×2) and a saturated sodium chloride solution (15 mL×2), and dried over anhydrous sodium sulfate for 4 h The reaction solution was subjected to vacuum filtration. A filtrate was concentrated under reduced pressure and purified by column chromatography (petroleum ether: ethyl acetate = 1:2) to obtain 480 mg of a white solid, namely a compound K44, with the yield of 57% in two steps. $^1H$ NMR (300 MHz, CDCl3) δ 8.44 (s, 1H), 7.35 (d, *J* = 7.8 Hz, 2H), 7.18 (d, J = 7.8 Hz, 2H), 6.79 (d, *J* = 4.7 Hz, 1H), 4.99 (s, 2H), 3.83 (s, 2H), 3.57 (s, 4H), 2.37 (s, 7H), 1.01 (s, 9H). 13C NMR (101 MHz, $CDCl_3$) δ 170.13, 162.74, 159.36, 141.25, 139.65, 134.38, 127.39, 127.24, 126.98, 116.22, 112.25, 58.07, 55.16, 54.67, 52.19, 47.50, 45.91, 41.93, 34.71, 28.62. HRMS(ESI) for $C_{23}H_{30}N_6O$ $[M+H]^+$: calcd, 407.2554; found, 407.2547.

**Example 45** 4-(((4-cyanopyrimidin-2-yl) (neopentyl)amino)methyl)-N-(2-(4-methylpiperazin-1-yl)-2-oxoethyl)benzamide (45)

Referring to the preparation steps of example 44, a white solid product with the yield of 34% was obtained. $^1H$ NMR (300 MHz, DMSO-d6) $\delta$ 8.72-8.47 (m, 2H), 7.77 (d, $J$ = 8.0 Hz, 2H), 7.23 (d, $J$ = 8.0 Hz, 2H), 7.18 (d, $J$ = 4.7 Hz, 1H), 4.94 (s, 2H), 3.59 (s, 2H), 3.18-3.02 (m, 6H), 2.95-2.80 (m, 2H), 2.70 (s, 3H), 1.89-1.69 (m, 3H), 0.96 (s, 9H). 13C NMR (101 MHz, $CDCl_3$) $\delta$ 167.71, 162.65, 159.41, 141.54, 141.10, 132.81, 127.62, 126.93, 116.25, 112.29, 58.21, 54.59, 52.36, 45.88, 44.51, 43.65, 34.67, 33.18, 28.56, 27.22. HRMS(ESI) for $C_{25}H_{34}N_6O$ $[M+H]^+$: calcd, 435.2867; found, 435.2859.

**Example 46 Inhibitory effect of compounds on Cat K**

Reagent information: Cat K inhibitor screening kit: batch number, 6L23K01500; supplier, BiVision. Drug preparation: compounds were dissolved in DMSO and prepared into 10 mM stock solutions. When in use, the stock solutions were prepared into the required concentrations by using a buffer.

Ref-03 was used as a comparative compound with the structural formula as follows:

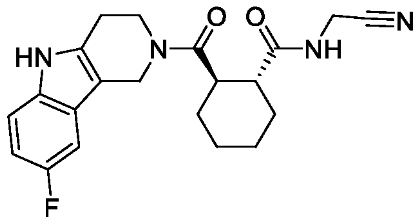

Ref- 03

.

**Experimental method**

1. Preliminary screening of drug

Cathepsin K (CTSK, EC 3.4.22.38) is a lysosomal cysteine protease, participates in osteoclastic bone remodeling and resorption, and can further degrade collagen, gelatin and elastin. The cathepsin K inhibitor screening kit from Biovision uses the ability of the active cathepsin K to cleave a synthetic AFC-based peptide substrate to release AFC and the AFC can be easily quantified by using a fluorometer or fluorescent microplate reader. In the presence of a specific cathepsin K specific inhibitor, the cleavage of the substrate is reduced/eliminated, resulting in a reduction or complete loss of AFC fluorescence. The simple and high-throughput adaptive assay kit can be used to screen/study/characterize a potential inhibitor of the cathepsin K.

CTSK Substrate-AFC —Cathepsin K→ Cleaved substrate + AFC (Fluorescence)

CTSK Substrate-AFC —Cathepsin K + CTSK inhibitor→ Decrease in fluorescence/No fluorescence

20 $\mu$L of a buffer, a Cat K inhibitor and test compounds (1 $\mu$M and 10 $\mu$M) were added to 96-well plates to serve as an EC well, an IC well and an S well, respectively; 50 $\mu$L of a Cat K enzyme solution was added into all the wells respectively and the mixture was incubated for 10-15 min at room temperature to establish an enzyme inhibitor complex; 30 $\mu$L of a Cat K substrate solution was added into all the wells and the mixture was incubated for 30-60 min at room temperature; and the final volume of the reaction was 100 $\mu$L. At 30-60 min, two time points T1 and T2 were selected to detect fluorescence absorption values (Ex/Em = 400/505 nm), the fluorescence absorption values were recorded as RFU1 and RFU2, and the inhibition rates (%) of the test compounds on the Cat K were calculated.

$$\text{Slope} = (\text{RFU2}-\text{RFU1})/(\text{T2}-\text{T1})$$

$$\text{Inhibition rate (\%)} = (\text{EC slope}-\text{S slope})/\text{EC slope}\times 100$$

2. $IC_{50}$ determination

The screening method was the same as 1.1, the concentrations of the test compounds were set between 0.1 nM and 10 μM, 4-5 concentrations were selected for detection, $IC_{50}$ curves were drawn, and $IC_{50}$ values were calculated.
Data processing: all the data were statistically analyzed using Graph pad.

**Experimental results**

The results were shown in Table 1.

Table 1 $IC_{50}$ (nM) of compounds

| **Compound No.** | Cat K $IC_{50}$ (nM) |
|---|---|
| K1 | B |
| K2 | C |
| K6 | C |
| K7 | C |
| K10 | C |
| K13 | A |
| K15 | A |
| K16 | C |
| K17 | C |
| K18 | B |
| K19 | B |
| K20 | B |
| K21 | B |
| K22 | B |
| Note: A, B and C represented activity ranges for $IC_{50}$ values, wherein A: < 10 nM, B: 10-100 nM and C: 100-500 nM. | |

The compounds of the present invention had a high inhibition rate on the Cat K, low $IC_{50}$ values and good inhibition activity on the Cat K. A part of the compounds such as K13 and K15 had the $IC_{50}$ values of less than 10 nM, and a part of the compounds such as K1 and K18-K22 had the $IC_{50}$ values of all less than 100 nM, such that the compounds of the present invention had better inhibitory effect on the Cat K.

**Example 47 Inhibitory effect of compounds on Cat B and Cat S**

**Screening of cathepsin B**

10 μL of a buffer, a Cat B inhibitor and test compounds (1 μM and 10 μM) were added to 96-well plates to serve as an EC well, an IC well and an S well, respectively; 50 μL of a Cat B enzyme solution was added into all the wells respectively and the mixture was incubated for 10-15 min at room temperature to establish an enzyme inhibitor complex; 40 μL of a Cat B substrate solution was added into all the wells and the mixture was incubated for 30-60 min at room temperature; and the final volume of the reaction was 100 μL. At 30-60 min, two time points T1 and T2 were selected to detect fluorescence absorption values (Ex/Em = 400/505 nm), the fluorescence absorption values were recorded as RFU1 and RFU2, and the inhibition rates (%) of the test compounds on the Cat B were calculated.

$$\text{Slope} = (\text{RFU2}-\text{RFU1})/(\text{T2}-\text{T1})$$

$$\text{Inhibition rate (\%)} = (\text{EC slope} - \text{S slope})/\text{EC slope} \times 100$$

$IC_{50}$ determination

The screening method was the same as above, the concentrations of the test compounds were set between 0.1

nM and 10 μM, 4-5 concentrations were selected for detection, $IC_{50}$ curves were drawn, and $IC_{50}$ values were calculated.

Data processing: all the data were statistically analyzed using Graph pad.

**Screening of cathepsin S**

10 μL of a buffer, a Cat S inhibitor and test compounds (1 μM and 10 μM) were added to 96-well plates to serve as an EC well, an IC well and an S well, respectively; 50 μL of a Cat B enzyme solution was added into all the wells respectively and the mixture was incubated for 10-15 min at room temperature to establish an enzyme inhibitor complex; 40 μL of a Cat S substrate solution was added into all the wells and the mixture was incubated for 30-60 min at room temperature; and the final volume of the reaction was 100 μL. At 30-60 min, two time points T1 and T2 were selected to detect fluorescence absorption values (Ex/Em = 400/505 nm), the fluorescence absorption values were recorded as RFU1 and RFU2, and the inhibition rates (%) of the test compounds on the Cat S were calculated.

$$\text{Slope} = (\text{RFU2}-\text{RFU1})/(\text{T2}-\text{T1})$$

$$\text{Inhibition rate (\%)} = (\text{EC slope}-\text{S slope})/\text{EC slope}\times 100$$

$IC_{50}$ determination

The screening method was the same as above, the concentrations of the test compounds were set between 0.1 nM and 10 μM, 4-5 concentrations were selected for detection, $IC_{50}$ curves were drawn, and $IC_{50}$ values were calculated.

Data processing: all the data were statistically analyzed using Graph pad.

The ratios of the inhibitory activities of the compounds against the Cat S and the Cat B to those against the Cat K were shown in Table 2.

Table 2 Ratios of inhibitory activities of part of compounds against Cat S and Cat B to those against Cat K

| **Compound No.** | Cat S/K | Cat B/K |
|---|---|---|
| K1 | > 800 | > 1,600 |
| K2 | >> 2,100 | >> 2,100 |
| K6 | >> 3,300 | >> 3,300 |
| K13 | >> 5,300 | >> 53,000 |
| K16 | >> 3,300 | >> 3,300 |
| K15 | ≈ 7,460 | > 15,000 |
| K13 | > 1,000 | > 1,000 |
| K18 | >> 5,780 | >> 5,780 |
| K21 | > 710 | > 710 |
| Odanacatib | < 290 | > 590 |

Note: ">>" indicated that at the test concentration, the inhibition rate did not exceed 20%; ">" indicated that at the test concentration, the inhibition rate was greater than 35%; "≈" indicated that at the test concentration, the inhibition rate was about 50%; and "<" indicated that at the tested concentration, the inhibition rate was less than 50%.

A part of the compounds showed 1,000-fold selectivity on the Cat K far more than that on the other two enzymes. Although the test values of a positive control Odanacatib in the experiment were slightly lower than those reported in the literature, compared with the positive control Odanacatib, the Cat K inhibitor of the present invention still obviously improved the selectivity on the Cat K and particularly made up for the defect of the selection capability on the Cat K and the Cat S.

**Example 48 Intrinsic clearance rate and half-life in rat liver microsomes of compounds**

**1. Preparation of sample solutions**

1 mL of a dichloromethane solution was respectively added into centrifuge tubes filled with test substance powders K2 (molecular formula C23H27N3O4, 4.6 mg), K3 (molecular formula C23H26FN3O4, 5.0 mg) and K4

(molecular formula C23H25F2N3O4, 5.6 mg) for dissolving, and subjected to ultrasonic treatment for 30 min to obtain a K2 stock solution with the mass concentration of 4.6 mg/mL, a K3 stock solution with the mass concentration of 5.0 mg/mL and a K4 stock solution with the mass concentration of 5.6 mg/mL. The stock solutions were sealedly stored in a refrigerator at -20°C for later use. Before use, the stock solutions were gradually diluted with methanol to corresponding mass concentration to respectively prepare 0.02, 0.05, 0.22, 0.49, 0.96, 2.37 and 4.90 ng/mL of K2 series standard sample working solutions, 0.02, 0.05, 0.10, 0.19, 1.00, 2.01 and 4.93 ng/mL K3 series standard sample working solutions, and 0.10, 0.20, 0.53, 0.96, 2.42, 5.10 and 10.10 ng/mL K4 series standard sample working solutions.

**2. Experimental method**

5 μL of the test drugs, 2 μL of rat liver microsomes and 468 μL of a PBS buffer solution were taken by using a pipette and prepared a premixed solution. The premixed solution was incubated for 5 min in a water bath kettle at 37°C, 47.5 μL of the premixed solution was taken after the incubation was finished, and the premixed solution, 100 μL of ice-cold acetonitrile and 2.5 μL of a PBS buffer solution were added to another new tube after to be gently mixed evenly to obtain a control group. The reaction was started by adding 22.5 μL of a NADPH solution to the remaining premixed solution, the reaction solution was gently pipetted, and the reaction was stopped by immediately taking 50 μL of the premixed solution and 100 μL of acetonitrile in a new tube. Then the incubation liquid was put into the water bath for incubation, 50 μL of the incubation liquid was respectively taken at 5 min, 10 min, 20 min, 30 min, 45 min, 60 min and 90 min, and the reaction was terminated by adding 100 μL of acetonitrile. The reaction solution was centrifuged at 12,000 r/min for 10 min, and the supernatant was taken and detected on a machine.

Chromatographic conditions:

Chromatographic column: Hypersil GOLD 100*2.1 mm 3 μm liquid chromatography column
Mobile phase: 0.1% formic acid aqueous solution (A); and 100% acetonitrile (B)
Gradient elution (0-1 min: 50%B, 1-5 min: 50%B-90%B, 5-7 min: 90%B, 7-7.1 min: 50%B, and 7.1-10 min: 50%B)
Flow rate: 0.3 ml/min; column temperature: 40°C; and injection volume: 5 μL
Mass spectrometry conditions
Electrospray ionization (ESI); ion spray voltage: 4,500 V; ion source temperature: 450°C; air curtain pressure: 35 psi; and declustering voltage: 80 V.

**3. Drawing of standard curves and investigation of quantitative lower limits**

Sample solutions were subjected to sample injection analysis under the chromatographic and mass spectrometry conditions, and the peak areas were recorded. Linear regression was performed by using the mass concentration of a test substance as an x-coordinate and the peak area of the test substance as a y-coordinate using a weighting method (weighting coefficient of 1/x2). The results showed that a linear range of K2 mass concentration detection was 0.02-4.90 ng/mL and a quantitative lower limit was 0.02 ng/mL; a linear range of K3 mass concentration detection was 0.02-4.93 ng/mL and a quantitative lower limit was 0.02 ng/mL; and a linear range of K4 mass concentration detection was 0.10-10.10 ng/mL and a quantitative lower limit was 0.10 ng/mL. Statistical analysis was performed on the basis of the results to calculate clearance rate ($Cl_{int}$) and half-life ($T_{1/2}$).

The results were shown in Table 3.

Table 3 Intrinsic clearance rate ($Cl_{int}$) and half-life ($T_{1/2}$) in rat liver microsomes of compounds

| **Compound No.** | $Cl_{int}$ | $T_{1/2}$ (h) |
|---|---|---|
| K20 | 180 | 0.777 |
| K21 | 190 | 0.706 |
| K22 | 90 | 1.475 |
| Ref-03* | > 250 | 0.6 |

Note: Data of Ref-03 clearance rate and half-life were taken from literature.

Compared with Ref-03, the compounds had reduced intrinsic clearance rate of the rat liver microsomes, wherein the clearance rate of the compound K4 was reduced by 2 times, the clearance rates of other compounds were all less than that of Ref-03. The half-life of Ref-03 was 0.6 h, while the half-life of the compounds of the present invention was all greater

than 0.6 h. Therefore, the compounds of the present invention were more metabolically stable.

**Example 49 Inhibitory effect of compounds on osteoclasts**

**1 Cell viability (cytotoxicity) assay**

RAW264.7 cells were inoculated at a density of $1\times10^5$/well in 96-well plates and cultured in DMEM containing 10% FBS and 1% penicillin/streptomycin. The cells were cultured in a constant-temperature incubator containing 5%CO2 at 37°C. After 24 h of the culture, different concentrations of inhibitor working solutions were respectively added to administration groups, and an equal volume of a PBS working solution was added to a control group. After the cells were continuously cultured for 2 days, a culture supernatant was removed, the cells were washed with PBS 3 times, and the viability of the cells was detected with a CCK8 cell proliferation detection kit. 100 μL of PBS containing CCK8 was added to each well, the mixture was incubated at 37°C in the dark for 2 h, the absorbance at 450 nm was detected by a microplate reader, and CCK8 was added to a culture medium containing free of cells and drug as a blank group.

The viability was calculated by the following equation:

Cell viability (%) = [(Average absorbance of administration group-average absorbance of blank wells)/(average absorbance of control group-average absorbance of blank group)]$\times$100.

The results were shown in Table 4.

Table 4 Effects of compounds on cytotoxicity to RAW264.7

| **Compound No.** | Viability % | |
|---|---|---|
| | 1 μM | 10 μM |
| K29 | 96.06% | 98.89% |
| K31 | 85.84% | 82.99% |
| Control group | 100% | |

It can be seen from table 5, the cell viabilities of RAW264.7 cells in a compound K29 at the concentrations of 1 μM and 10 μM were 96.06% and 98.89%, respectively; the cell viabilities of RAW264.7 cells in a compound K31 at the concentrations of 1 μM and 10 μM were 85.84% and 82.99%, respectively. It can be seen that the compound K29 had no significant cytotoxicity to RAW264.7 cells at the concentrations of 1 μM and 10 μM, and the compound K31 had cytotoxicity to RAW264.7 cells at the concentrations of 1 μM and 10 μM.

**2 Osteoclast differentiation**

RAW264.7 cells were inoculated at a density of $1\times10^5$/well in 96-well plates and cultured in DMEM containing 10% fetal bovine serum and 1% penicillin/streptomycin. The cells were cultured in a constant-temperature incubator containing 5%CO2 at 37°C. 100 ng/mL of a RANKL inducing solution was added into each well of the administration groups and a model group to culture osteoclasts, the solution (containing RANKL) was changed every 2-3 days, inhibitor working solutions with different concentrations were added into the administration groups after the solution was changed for the 3rd time, a PBS working solution with the same volume was added into the model group, and the cells were continuously cultured for 24 h; and in the control group, the RANKL inducing solution and the inhibitor working solution were not added, and the PBS working solution with the same total volume was added. A culture supernatant was removed, the cells were rinsed 3 times with PBS and stained with a TRAP staining kit, and the multinucleated osteoclasts were observed under an optical microscope and counted.

The results were shown in Table 5.

Table 5 Effects of compounds on number of osteoclasts after differentiation of RAW264.7 cells induced by RANKL

| **Compound No.** | Concentration | Number of osteoclasts |
|---|---|---|
| K29 | 1 μM | 13 |
| | 10 μM | 11.25 |
| K31 | 100 nM | 13.25 |
| | 500 nM | 14 |

(continued)

| Compound No. | Concentration | Number of osteoclasts |
|---|---|---|
| Model group | - | 19.50 |
| Control group | - | - |

It can be seen from Table 5 that compared with the model group, the compound K29 at the concentrations of 1 μM and 10 μM and the K31 at the concentrations of 100 nM and 500 nM both had a tendency to inhibit osteoclastogenesis, wherein the K29 obviously inhibited the osteoclastogenesis at the concentration of 10 μM. Combining Tables 4 and 5, the compound K29 was not cytotoxic and had a good osteoclastogenesis inhibitory effect.

**Example 50 *In-vivo* toxicity study**

Male ICR mice of 6-8 weeks were used as experimental subjects and randomly divided according to body weight after adaptive feeding for one week. Then compounds of different concentrations were prepared and used for single-administration and multiple-administration experiments by intraperitoneal injection (i.p), and a vehicle of the same component was used as a reference.

As shown in FIG. 1, in the case of single administration of 500 mg/kg and 300 mg/kg, no death phenomenon or obvious toxic reaction occurred in the mice observed on day 10. During this period, the body weight of the mice was measured daily. No significant difference was shown in the body weight between administration groups and a vehicle group (all $P > 0.05$). In the multiple-administration experiment, the administration doses of 50 mg/kg and 25 mg/kg were selected for administration at the frequency of once a day. No death phenomenon or obvious toxic reaction occurred in the mice after one week of continuous administration and no death and no obvious toxic reaction occurred. No significant difference was shown in the body weight between the administration groups and the vehicle group (all $P > 0.05$). As shown in FIG. 2, a histopathological study was performed on the mice in the single-administration and multiple-administration maximum dose groups. Compared with the vehicle group, no obvious pathological change was observed in the mice treated with a compound K18. 50 mg/kg and 25 mg/kg as safe administration doses met the safety requirements of subsequent in-vivo pharmacodynamics studies.

The above results showed that the compound K18 had relatively low in-vivo toxicity and a certain safety.

**Example 51 *In-vivo* efficacy experiment against osteoporosis**

An osteoporosis mouse model was established by injecting dexamethasone subcutaneously into male C57BL/6 mice, 8 days after the model establishment, the model mice were administered intraperitoneally at administration doses of 12 mg/kg, 24 mg/kg and 36 mg/kg, and a Cat K inhibitor Ballicaib was used as a positive control. After 6 weeks of continuous administration once a day, the mice were anesthetized and dissected, blood serum was retained, the content of a bone resorption biomarker CTX in the blood was detected by ELISA, and one side of the femur was taken for measuring the bone density. The therapeutic effects of the Cat K inhibitors on osteoporosis were evaluated by comparison with the vehicle group. The effects of the preliminary experiment were obvious. At present, formal experiments were performed.

**Example 52 *In-vivo* efficacy experiment against bone metastasis**

The treatment effects of the compounds of the present invention on bone metastasis were further researched by establishing a prostate cancer bone metastasis model by injecting C4-2B cells into the tibia of NOD-SCID mice by puncture. After 4 weeks of molding, the model mice were administered intraperitoneally at administration doses of 25 mg/kg and 50 mg/kg, and a common anti-bone metastasis drug zoledronic acid was used as a positive control. After 4 weeks of continuous administration once a day, the changes of body weight and tumor volume of the mice were observed and recorded during the period. After the administration, the mice were sacrificed. The content of prostate specific antigen (PSA) in plasma was tested by ELISA, then the tibial bone density of the mice was tested, and pathological sections were made to test the pathological changes of bone tissues. From these test results, the inhibitory effect on the development of prostate cancer bone metastasis and the protection effect on the bone tissues by the Cat K inhibitors were evaluated.

The test results preliminarily showed that the compound K18 can inhibit the migration, invasion and adhesion processes of the C4-2B cells in a concentration-dependent manner, indicating that the Cat K inhibitors can effectively exert the antitumor activity by influencing the metastasis process of tumors.

**Example 53 Experiment of absolute bioavailability of oral administration in rats**

1 Instruments

Acquity ultra performance liquid chromatography (UPLC) system (Waters corporation); TSQ Endura triple quadrupole MS instrument (Thermo corporation); KQ-500E type ultrasonic cleaner (Kunshan Ultrasonic Instrument Co., Ltd); 3K15 benchtop high-speed centrifuge (SIGMA, Germany); and XW-80A vortex mixer (Shanghai Jingke Industrial Co., Ltd.)

2 Drugs and reagents

Test substances: K29 (molecular weight of 439.21 and mass of 27 mg) and K31 (molecular weight of 457.20 and mass of 14 mg+20 mg).

Reagents: HS-15 was analytically pure, sodium carboxymethylcellulose (CMC-Na) was medicinal grade, formic acid, methanol and acetonitrile were chromatographic grade, and water was purified water.

3 Experimental method

3.1 Chromatographic and mass spectrometry conditions

3.1.1 Chromatographic conditions

Chromatographic column: Thermo Scientific 100*2.1 mm 3 μm liquid chromatography column
Mobile phase: 5 mM ammonium acetate aqueous solution (A); and 100% acetonitrile (B)
Gradient elution (0-0.5 min: 20%B, 0.5-1.5 min: 20%-90%B, 1.5-4 min: 90%B, 4-4.1 min: 90%-20%B, and 4.1-4.5 min: 20%B)
Flow rate: 0.25 ml/min; column temperature: 40°C; and injection volume: 2 μL

3.1.2 Mass spectrometry conditions

Electrospray ionization (ESI); ion spray voltage: 3,500 V; ion source temperature: 300°C; auxiliary gas: 45 Arb; and sheath gas: 10 Arb.

3.2 Preparation of administration solutions

Intravenous intraperitoneal administration solution: prepared with 0.5% HS-15 normal saline solution with the concentration of 0.2 mg/mL; and
intragastric administration suspension: ground and suspended with 0.5% CMC-Na aqueous solution with the concentration of 0.5 mg/mL.

3.3 Animal administration and sample treatment

3.3.1 Administration and blood sampling

Each administration was tested using 2 SD rats per compound.

The two compounds K29 and K31 were subjected to intravenous injection and intragastric administration respectively. The intravenous administration volume was 5 mL/kg. 0.2 mL of blood was collected in jugular veins 2 min, 10 min, 30 min, 1 h, 2 h, 4 h, 7.5 h and 24 h after the administration respectively. The volume was 10 mL/kg in the intragastric administration group. 0.2 mL of blood was collected in jugular veins 5 min, 15 min, 30 min, 1 h, 2 h, 4 h, 8 h and 24 h after the administration respectively. All blood samples were anticoagulated by heparin and centrifuged for 10 min at 4,000 g. The supernatant plasma was taken for cryopreservation to be tested.

3.3.2 Sample treatment

50 μL of the plasma sample was taken, 150 μL of acetonitrile was added, the mixture was evenly mixed by vortex and centrifuged at 9,100 g for 10 min, and a supernatant was taken and 2 μL of the supernatant was injected and detected on a machine.

The experimental results were shown in Tables 6-10.

Table 6 Data of plasma concentration (ng/mL) of K29 in SD rats after K29 administration

| 1 mg/kg for intravenous administration | | | 5 mg/kg for intragastric administration | | |
|---|---|---|---|---|---|
| Time point (h) | 1 | 2 | Time point (h) | 5 | 6 |
| 0.033 | 1,047 | 1,406 | 0.083 | 512 | 458 |
| 0.17 | 502 | 1,292 | 0.25 | 1,136 | 1,434 |
| 0.5 | 420 | 698 | 0.5 | 973 | 1,412 |
| 1 | 250 | 346 | 1 | 822 | 997 |
| 2 | 65.2 | 106 | 2 | 518 | 631 |
| 4 | 10.1 | 22.3 | 4 | 268 | 204 |
| 6 | 2.73 | 7.34 | 6 | 97.3 | 73.2 |
| 7.5 | 1.40 | 4.24 | 7.5 | 68.5 | 48.6 |
| 24 | BLQ | BLQ | 24 | BLQ | BLQ |

Table 7 Data of plasma concentration (ng/mL) of K29 in rats after K29 administration

| Parameter | Unit | Intravenous administration | | | Intragastric administration | | |
|---|---|---|---|---|---|---|---|
| | | No1 | No2 | Mean | No1 | No2 | Mean |
| $AUC_{(0-t)}$ | μg/L*h | 707 | 1,216 | 961 | 2,817 | 3,153 | 2,985 |
| $AUC_{(0-\infty)}$ | μg/L*h | 708 | 1,220 | 964 | 2,997 | 3,257 | 3,127 |
| $MRT_{(0-t)}$ | h | 0.864 | 0.869 | 0.8665 | 2.119 | 1.795 | 1.957 |
| $MRT_{(0-\infty)}$ | h | 0.874 | 0.894 | 0.884 | 2.6 | 2.047 | 2.3235 |
| $t_{1/2z}$ | h | 0.874 | 1.038 | 0.956 | 1.812 | 1.49 | 1.651 |
| Tmax | h | 0.033 | 0.033 | 0.033 | 0.25 | 0.25 | 0.25 |
| $CL_z$ | L/h/kg | 1.41 | 0.82 | 1.12 | 1.67 | 1.54 | 1.60 |
| $V_z$ | L/kg | 1.78 | 1.23 | 1.50 | 4.36 | 3.3 | 3.83 |
| $C_{max}$ | μg/L | 1,047 | 1,406 | 1,226.5 | 1,136 | 1,434 | 1,285 |
| F | % | | | | | | 62.1 |

Table 8 Pharmacokinetic parameters of K31 in plasma of SD rats after K31 administration

| 1 mg/kg for intravenous administration | | | 5 mg/kg for intragastric administration | | |
|---|---|---|---|---|---|
| Time point (h) | 1 | 2 | Time point (h) | 5 | 6 |
| 0.033 | 1,813 | 1,231 | 0.083 | 463 | 774 |
| 0.17 | 847 | 948 | 0.25 | 783 | 1,089 |
| 0.5 | 530 | 664 | 0.5 | 750 | 918 |
| 1 | 305 | 411 | 1 | 643 | 804 |
| 2 | 110 | 174 | 2 | 515 | 529 |
| 4 | 20.9 | 37.6 | 4 | 235 | 258 |
| 6 | 4.06 | 10.6 | 6 | 108 | 111 |
| 7.5 | 1.90 | 4.90 | 7.5 | 79.0 | 68.7 |
| 24 | BLQ | BLQ | 24 | BLQ | 8.63 |

Table 9 Pharmacokinetic parameters of K31 in plasma of SD rats after K31 administration

| Parameter | Unit | Intravenous administration | | | Intragastric administration | | |
|---|---|---|---|---|---|---|---|
| | | No1 | No2 | Mean | No1 | No2 | Mean |
| $AUC_{(0-t)}$ | μg/L*h | 1,043 | 1,289 | 1,166 | 2,475 | 3,464 | 2,970 |
| $AUC_{(0-\infty)}$ | μg/L*h | 1,044 | 1,294 | 1,169 | 2,629 | 3,465 | 3,047 |
| $MRT_{(0-t)}$ | h | 0.89 | 1.12 | 1.01 | 2.29 | 3.46 | 2.88 |
| $MRT_{(0-\infty)}$ | h | 0.9 | 1.15 | 1.03 | 2.75 | 3.47 | 3.11 |
| $t_{1/2z}$ | h | 0.84 | 0.959 | 0.900 | 1.78 | 1.86 | 1.82 |
| $T_{max}$ | h | 0.033 | 0.033 | 0.033 | 0.25 | 0.25 | 0.25 |
| $CL_z$ | L/h/kg | 0.957 | 0.773 | 0.865 | 1.902 | 1.443 | 1.67 |
| $V_z$ | L/kg | 1.161 | 1.07 | 1.12 | 4.87 | 3.88 | 4.38 |
| $C_{max}$ | μg/L | 1,813 | 1,231 | 1,522 | 783 | 1,089 | 936 |
| F | % | | | | | | 51.0 |

Table 10 Conclusion of PK test results of oral administration or intraperitoneal injection of each compound in rats

| Compound | Mode of administration | Bioavailability | $t_{1/2z}$ | $T_{max}$ | $CL_z$ | $V_z$ | $C_{max}$ |
|---|---|---|---|---|---|---|---|
| | | % | h | h | L/h/kg | L/kg | ng/mL |
| K29 | Oral administration | 62.1 | 1.65 | 0.25 | 1.60 | 3.83 | 1,285 |
| K31 | Oral administration | 51.0 | 1.82 | 0.25 | 1.67 | 4.38 | 936 |

It can be seen from Table 10, the oral administration bioavailability of the compounds K29 and K31 was 62.1% and 51.0% respectively. The oral administration bioavailability was good.

## Claims

1. A compound of formula 0 or a pharmaceutically acceptable salt thereof or an optical isomer thereof, wherein the general structure thereof is as follows:

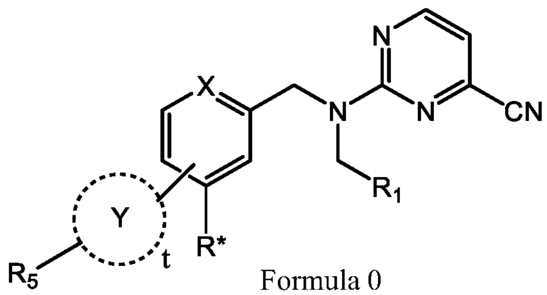

Formula 0

wherein X is C or N;
$R_1$ is selected from H, substituted or unsubstituted C1-10 alkyl, and substituted or unsubstituted C3-C10 cycloalkyl; wherein the substituent is selected from halogen, amino, cyano, hydroxyl, an aldehyde group, carboxyl and sulfonyl;
R* is selected from halogen or C1-6 alkyl;
Y is a ring group, located at any position of an attached aromatic ring, attached to the aromatic ring through 1 or 2 carbon atoms, and optionally C3-10 cycloalkyl, a C6-12 aromatic ring or a C5-12 heterocyclic ring;
wherein the C6-12 aromatic ring contains a C6-12 aromatic ring or a C6-12 heteroaromatic ring; wherein the C6-12 heteroaromatic ring contains at least one heteroatom; wherein the C5-12 heterocyclic ring is a saturated heterocyclic ring or an unsaturated heterocyclic ring, and the heterocyclic ring contains 1-3 heteroatoms; wherein the heteroatom is optionally O, N or S;
t is 0 or 1; when t is 0, $R_5$ is located at any position of the attached aromatic ring; and
$R_5$ is selected from H, halogen, amino, cyano, C1-10 alkyl, C1-10 alkoxy, C3-10 cycloalkyl, substituted C3-10 heterocycloalkyl, -S(O) $2R_2$, $-C(O)R_2$, $-NR_3R_4$, $-C(O)NHR_7$, - $SR_6$ and $-OR_6$;

wherein the substituent of the C3-10 heterocycloalkyl is selected from hydroxyl and C1-10 alkyl;
wherein the C1-10 alkyl may be further substituted by hydroxy or -C(O)$R_2$;
wherein $R_2$ is selected from H, amino, halogen, substituted or unsubstituted C1-6 alkyl, C1-6 alkoxy, C3-8 cycloalkyl and C3-8 heterocycloalkyl; wherein the substituent of the C1-6 alkyl, the C1-6 alkoxy, the C3-8 cycloalkyl or the C3-8 heterocycloalkyl is C1-6 alkyl;
wherein $R_3$ and $R_4$ are each independently selected from H and -C(O)$R_8$; or $R_3$ and $R_4$ together with N to which they are attached form a 4-8 membered ring containing at least one N; wherein $R_8$ is selected from C1-6 alkyl substituted by piperazinyl or methylpiperazinyl;
wherein $R_6$ is selected from C1-6 alkyl;
wherein $R_7$ is selected from C1-6 alkyl, wherein the C1-6 alkyl may be further substituted by -C(O)$R_9$, wherein $R_9$ is selected from piperazinyl or methylpiperazinyl;
wherein the C3-10 heterocycloalkyl or the C3-8 heterocycloalkyl contains 1-3 heteroatoms, and the heteroatom is optionally O, N or S; and
wherein the halogen is mono-substituted or poly-substituted and selected from F, Cl, Br and I.

**2.** A compound of formula I or formula II or a pharmaceutically acceptable salt thereof or an optical isomer thereof, wherein the general structure thereof is as follows:

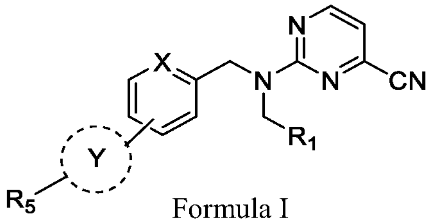

Formula I

or

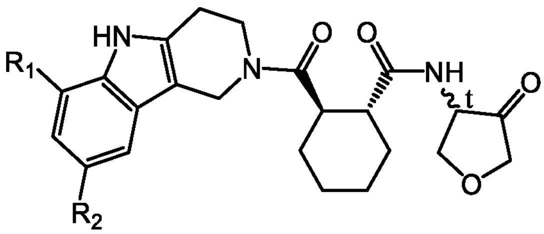

Formula II

wherein in formula I, X is C or N;
$R_1$ is selected from H, substituted or unsubstituted C1-10 alkyl, and substituted or unsubstituted C3-C10 cycloalkyl; wherein the substituent is selected from halogen, amino, cyano, hydroxyl, an aldehyde group, carboxyl and sulfonyl;
Y is a ring group, located at any position of an attached aromatic ring, and optionally C3-10 cycloalkyl, a C6-12 aromatic ring or a C5-12 heterocyclic ring,
wherein the C6-12 aromatic ring contains a C6-12 aromatic ring or a C6-12 heteroaromatic ring; wherein the C6-12 heteroaromatic ring contains at least one heteroatom; wherein the C5-12 heterocyclic ring is a saturated heterocyclic ring or an unsaturated heterocyclic ring, and the heterocyclic ring contains 1-3 heteroatoms; wherein the heteroatom is optionally O, N or S;
$R_5$ is selected from H, halogen, amino, cyano, C1-10 alkyl, C1-10 alkoxy, C3-10 cycloalkyl, -S(O) 2$R_2$, -C(O)$R_2$, -N$R_3R_4$, -S$R_6$ and -O$R_6$; wherein $R_2$ is selected from H, amino, halogen, C1-6 alkyl, C1-6 alkoxy and C3-8 cycloalkyl; wherein $R_3$ and $R_4$ together with N to which they are attached form a 4-8 membered ring containing at least one N; wherein $R_6$ is selected from C1-6 alkyl; and
wherein the halogen is mono-substituted or poly-substituted and selected from F, Cl, Br and I; and
in formula II, $R_1$ and $R_2$ are each independently selected from H, halogen, cyano, amino, substituted or unsubstituted C1-6 alkyl, substituted or unsubstituted C1-6 alkoxy, and the substituted C1-6 alkyl or C1-6 alkoxy is further substituted by at least one halogen or hydroxyl; and t in formula II is a chemical bond, optionally at least one of 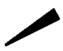, 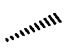 or 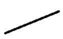.

**3.** The compound or the pharmaceutically acceptable salt thereof or the optical isomer thereof according to claim 2, wherein in formula I,

X is C or N;
$R_1$ is selected from C1-10 alkyl and C3-C10 cycloalkyl;
Y is a ring group, optionally C3-10 cycloalkyl, a C6-12 aromatic ring or a C5-12 heterocyclic ring;
wherein the C6-12 aromatic ring contains a C6-12 aromatic ring or a C6-12 heteroaromatic ring; wherein the C6-12 heteroaromatic ring contains at least one heteroatom; wherein the C5-12 heterocyclic ring is a saturated heterocyclic ring or an unsaturated heterocyclic ring, and the heterocyclic ring contains 1-3 heteroatoms; wherein

the heteroatom is optionally O, N or S;
$R_5$ is selected from H, halogen, amino, cyano, C1-10 alkyl, C1-10 alkoxy, C3-10 cycloalkyl, $-S(O)_2R_2$, -C(O) $2R_2$, $-NR_3R_4$, $-SR_6$ and $-OR_6$; wherein $R_2$ is selected from H, amino, halogen, C1-6 alkyl, C1-6 alkoxy and C3-8 cycloalkyl; wherein $R_3$ and $R_4$ together with N to which they are attached form a 4-8 membered ring containing at least one N; wherein $R_6$ is selected from C1-6 alkyl;
wherein the halogen is mono-substituted or poly-substituted and selected from F, Cl, Br and I; and
in formula I, $R_1$ and $R_2$ are each independently selected from H, halogen, substituted or unsubstituted C1-3 alkyl, and substituted or unsubstituted C1-3 alkoxy, and the substituted C1-3 alkyl or C1-3 alkoxy is further substituted by at least one F.

**4.** The compound or the pharmaceutically acceptable salt thereof or the optical isomer thereof according to claim 2, wherein Y is selected from the following groups: in formula I,

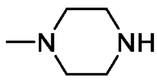,

phenyl, pyridyl, thienyl, thiazolyl; and in formula II, $R_1$ and $R_2$ are each independently selected from H, F, Cl, Br, methyl, methoxyl and trifluoromethoxy.

**5.** The compound or the pharmaceutically acceptable salt thereof or the optical isomer thereof according to claim 2, wherein in formula I, $R_5$ is selected from H, F, Cl, cyano, methyl, methylthio, methoxy, methylsulfonyl, methylcarbonyl and methylpiperazinyl; and in formula II, $R_1$ and $R_2$ are each independently selected from H and F.

**6.** A compound of formula 0 or formula II or a pharmaceutically acceptable salt thereof or an optical isomer thereof, specifically selected from the following compounds:

1) 2-[(2,2-dimethylpropyl){[4-(4-methylpiperazin-1-yl)phenyl]methyl} amino]pyrimidine-4-carbonitrile
2) 2-[(2,2-dimethylpropyl)[(4-phenylphenyl)methyl]amino]pyrimidine-4-carbonitrile
3) 2-[(2,2-dimethylpropyl) ({4-[4-(methylthio)phenyl]phenyl}methyl)amino]pyrimidine-4-carbonitrile
4) 2-[(2,2-dimethylpropyl) {[4-(4-fluorophenyl)phenyl]methyl} amino]pyrimidine-4-carbonitrile
5) 2-[(2,2-dimethylpropyl){[4-(4-methoxyphenyl)phenyl]methyl} amino]pyrimidine-4-carbonitrile
6) 2-[(2,2-dimethylpropyl) ({4-[4-(methylsulfonyl)phenyl]phenyl}methyl)amino]pyrimidine-4-carbonitrile
7) 2-({[4-(5-cyanothiophen-2-yl)phenyl]methyl}(2,2-dimethylpropyl)amino)pyrimidine-4-carbonitrile
8) 2-({[4-(6-chloropyridin-3-yl)phenyl]methyl}(2,2-dimethylpropyl)amino)pyrimidine-4-carbonitrile
9) 2-({[4-(3,4-dichlorophenyl)phenyl]methyl}(2,2-dimethylpropyl)amino)pyrimidine-4-carbonitrile
10) 2-[(2,2-dimethylpropyl){[4-(5-methylthiophen-2-yl)phenyl]methyl}amino]pyrimidine-4-carbonitrile
11) 4-(4-{[(4-cyanopyrimidin-2-yl) (2,2-dimethylpropyl)amino]methyl}phenyl)methyl benzoate
12) 2-({[4-(4-chloro-3-fluorophenyl)phenyl]methyl}(2,2-dimethylpropyl)amino)pyrimidine-4-carbonitrile
13) 2-[(2,2-dimethylpropyl) ({4-[4-(4-methylpiperazin-1-yl)phenyl]phenyl} methyl)amino]pyrimidine-4-carbonitrile
14) 2-[(cyclohexylmethyl)[(4-phenylphenyl)methyl]amino]pyrimidine-4-carbonitrile
15) 2-[(cyclohexylmethyl) ({4-[4-(4-methylpiperazin-1-yl)phenyl]phenyl} methyl)amino]pyrimidine-4-carbonitrile
16) 2-[(cyclohexylmethyl) ({4-[4-(methylsulfonyl)phenyl]phenyl} methyl)amino]pyrimidine-4-carbonitrile
17) 2-(neopentyl(4-(thiazol-4-yl)benzyl)amino)pyrimidine-4-carbonitrile
18) 2-((4-(2-(4-methylpiperazin-1-yl)thiazol-4-yl)benzyl) (neopentyl)amino)pyrimidine-4-carbonitrile
19) (1R,2R)-2-(8-fluoro-2,3,4,9-tetrahydro-1H-pyrido[3,4-b]indole-2-carbonyl)-N-(4-oxotetrahydrofuran-3-yl) cyclohexane-1-carboxamide;
20) (1R,2R)-2-(2,3,4,5-tetrahydro-1H-pyrido[4,3-b]indole-2-carbonyl)-N-(4-oxotetrahydrofuran-3-yl)-cyclohexane-1-carboxamide;
21) (1R,2R)-2-(8-fluoro-2,3,4,9-tetrahydro-1H-pyrido[3,4-b]indole-2-carbonyl)-N-((S)-4-oxotetrahydrofuran-3-yl)cyclohexane-1-carboxamide;
22) (1R,2R)-2-(6,8-difluoro-2,3,4,9-tetrahydro-1H-pyrido[3,4-b]indole-2-carbonyl)-N-((S)-4-oxotetrahydrofuran-3-yl)cyclohexane-1-carboxamide;
23) (1R,2R)-2-(8-chloro-2,3,4,5-tetrahydro-1H-pyrido[4,3-b]indole-2-carbonyl)-N-((S)-4-oxotetrahydrofuran-3-yl)cyclohexane-1-carboxamide;
24) (1R,2R)-2-(8-bromo-2,3,4,5-tetrahydro-1H-pyrido[4,3-b]indole-2-carbonyl)-N-((S)-4-oxotetrahydrofuran-3-yl)cyclohexane-1-carboxamide;

25) (1R,2R)-2-(6-(trifluoromethoxy)-2,3,4,5-tetrahydro-1H-pyrido[4,3-b]indole-2-carbonyl)-N-(4-oxotetrahydrofuran-3-yl)-cyclohexane-1-carboxamide;
26) (1R,2R)-2-(6-(trifluoromethoxy)-2,3,4,5-tetrahydro-1H-pyrido[4,3-b]indole-2-carbonyl)-N-(S)-(4-oxotetrahydrofuran-3-yl)-cyclohexane-1-carboxamide;
27) (1R,2R)-2-(6-fluoro-2,3,4,5-tetrahydro-1H-pyrido[4,3-b]indole-2-carbonyl)-N-(S)-(4-oxotetrahydrofuran-3-yl)-cyclohexane-1-carboxamide;
28) (1R,2R)-2-(6-fluoro-2,3,4,5-tetrahydro-1H-pyrido[4,3-b]indole-2-carbonyl)-N-(S)-(4-oxotetrahydrofuran-3-yl)-cyclohexane-1-carboxamide;
29) (1R,2R)-2-(6-methoxy-2,3,4,5-tetrahydro-1H-pyrido[4,3-b]indole-2-carbonyl)-N-(S)-(4-oxotetrahydrofuran-3-yl)-cyclohexane-1-carboxamide;
30) (1R,2R)-2-(6-methoxy-2,3,4,5-tetrahydro-1H-pyrido[4,3-b]indole-2-carbonyl)-N-(4-oxotetrahydrofuran-3-yl)-cyclohexane-1-carboxamide;
31) (1R,2R)-2-(8-fluoro-6-methoxy-2,3,4,5-tetrahydro-1H-pyrido[4,3-b]indole-2-carbonyl)-N-(S)-(4-oxotetrahydrofuran-3-yl)-cyclohexane-1-carboxamide;
32) (1R,2R)-2-(8-fluoro-6-methoxy-2,3,4,5-tetrahydro-1H-pyrido[4,3-b]indole-2-carbonyl)-N-(4-oxotetrahydrofuran-3-yl)-cyclohexane-1-carboxamide;
33) 2-((4-chlorobenzyl) (neopentyl)amino)pyrimidine-4-carbonitrile;
34) 2-((4-bromobenzyl) (neopentyl)amino)pyrimidine-4-carbonitrile;
35) 2-((naphthalen-2-ylmethyl) (neopentyl)amino)pyrimidine-4-carbonitrile;
36) 2-(neopentyl(quinolin-2-ylmethyl)amino)pyrimidine-4-carbonitrile;
37) 2-(((4'-(4-(2-hydroxyethyl)piperazin-1-yl)-[1,1'-biphenyl]-4-yl)methyl) (neopentyl)amino)pyrimidine-4-carbonitrile;
38) 2-(((4'-(4-hydroxypiperidin-1-yl)-[1,1'-biphenyl]-4-yl)methyl) (neopentyl)amino)pyrimidine-4-carbonitrile;
39) 2-(((2-methyl-4' -(4-methylpiperazin-1 -yl)- [1,1' -biphenyl]-4-yl)methyl) (neopentyl)amino)pyrimidine-4-carbonitrile;
40) 2-(((2-chloro-4' -(4-methylpiperazin-1 -yl)- [1,1' -biphenyl]-4-yl)methyl) (neopentyl)amino)pyrimidine-4-carbonitrile;
41) 2-(neopentyl(4-(4-(2-oxopropyl)piperazin-1-yl)benzyl)amino)pyrimidine-4-carbonitrile;
42) 2-(isobutyl((4'-(4-methylpiperazin-1-yl)-[1,1'-biphenyl]-4-yl)methyl)amino)pyrimidine-4-carbonitrile;
43) N-(4-((4-cyanopyrimidin-2-yl) (neopentyl)amino)methyl)phenyl)-2-(4-methylpiperazin-1-yl)acetamide;
44) 2-((4-(4-methylpiperazin-1-carbonyl)benzyl) (neopentyl)amino)pyrimidine-4-carbonitrile; and
45) 4-(((4-cyanopyrimidin-2-yl) (neopentyl)amino)methyl)-N-(2-(4-methylpiperazin-1-yl)-2-oxoethyl)benzamide.

**7.** A method for preparing the compound of formula I or formula II according to claim 2, wherein the method for preparing the compound of formula I comprises the following steps:

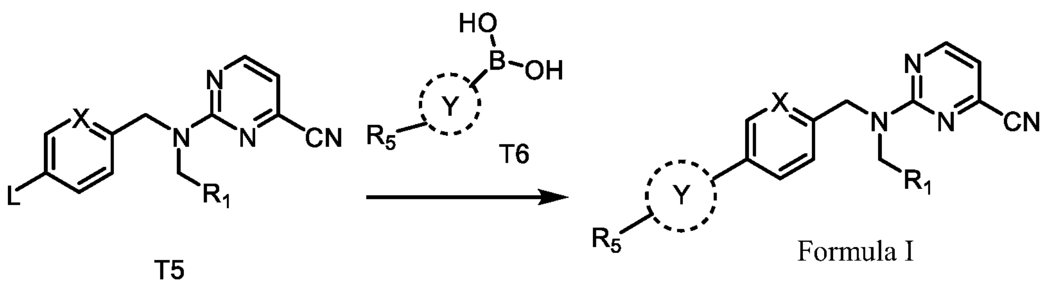

L is halogen reacting a compound T5 with a compound T6 to generate the compound of formula I, wherein $R_1$, $R_5$, X and Y are as defined in claim 1;
and/or, the method for preparing the compound of formula II comprises the following steps:

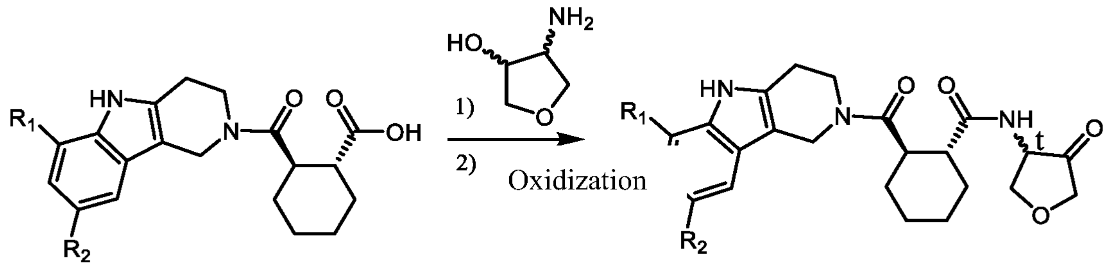


P2

Formula II

step 1), subjecting a compound P2 and 4-aminotetrahydrofuran-3-ol to condensation reaction; and
step 2), oxidizing the product obtained in step 1 by an oxidant to generate the compound of formula II.

**8.** The preparation method according to claim 7, wherein a method for preparing the compound T5 is as follows:

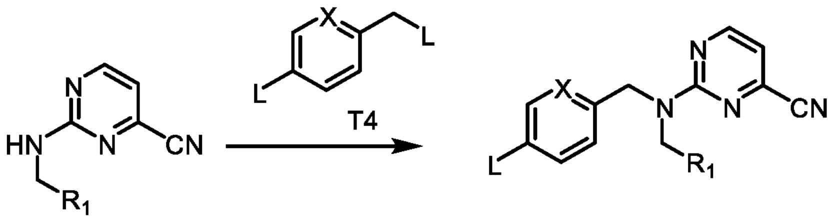

T3

T4

T5

reacting a compound T3 with a compound T4 to generate the compound T5.

**9.** The method according to claim 7, wherein a method for synthesizing the compound P2 is as follows:

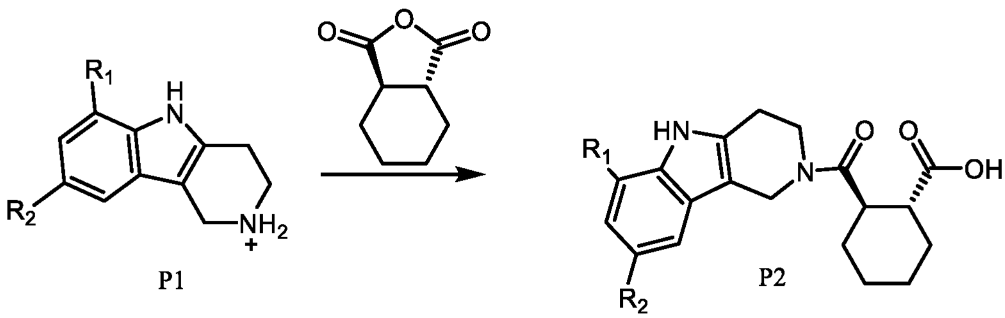

P1

P2

reacting a compound P1 with (3aR,7aS)-hexahydroisobenzofuran-1,3-dione to generate the compound P2.

**10.** A pharmaceutical composition containing the compound or the pharmaceutically acceptable salt thereof or the optical isomer thereof according to any one of claims 1-6.

**11.** Use of the compound or the pharmaceutically acceptable salt thereof or the optical isomer thereof according to any one of claims 1-6 in the preparation a drug for treating a disease taking cathepsin K as a target.

**12.** The use according to claim 11, wherein the disease taking cathepsin K as a target comprises a tumor, a thyroid diseases, a cardiovascular disease, a bone disease and a gum disease; preferably, the thyroid disease comprises hyperthyroidism; preferably, the cardiovascular disease comprises atherosclerosis, cardiac hypertrophy and heart failure; preferably, the bone disease comprises osteoporosis, osteoarthritis and rheumatoid arthritis; preferably, the gum disease comprises gingivitis and periodontitis; and preferably, the disease taking cathepsin K as a target is osteoporosis.

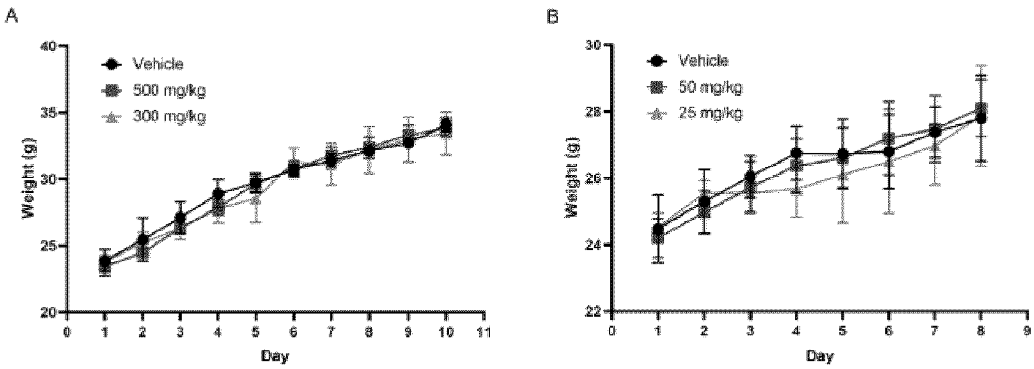
A
Vehicle
500 mg/kg
300 mg/kg
Weight (g)
Day
B
Vehicle
50 mg/kg
25 mg/kg
Weight (g)
Day

FIG. 1

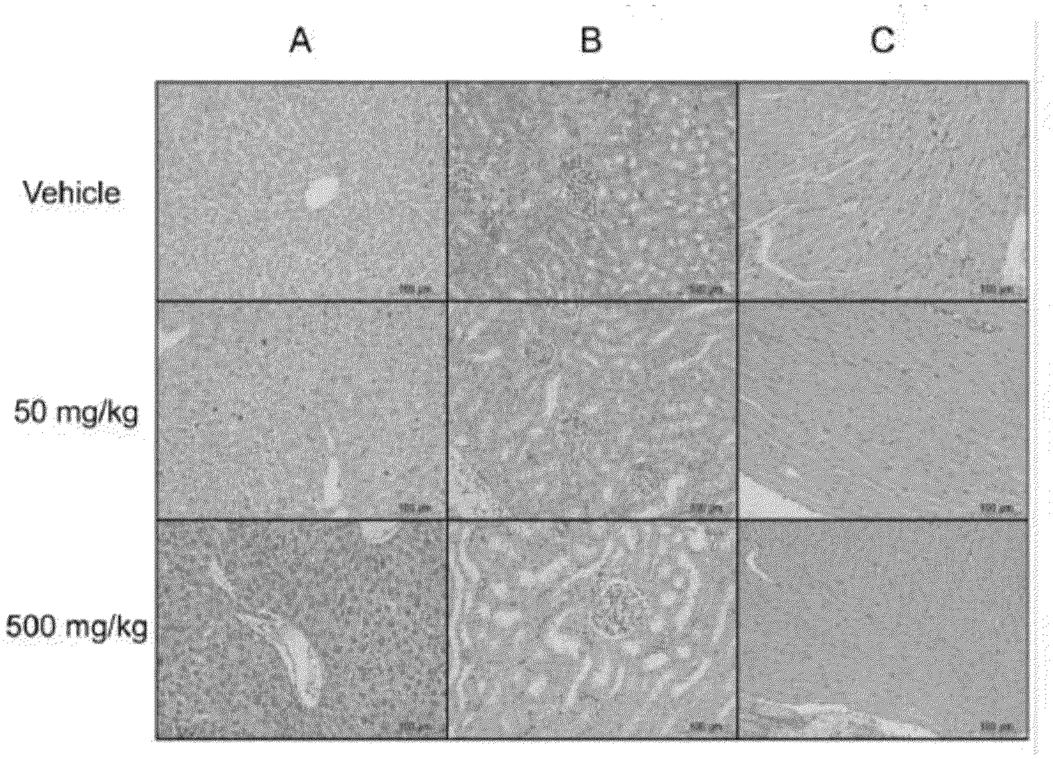
A
B
C
Vehicle
50 mg/kg
500 mg/kg

FIG. 2

**INTERNATIONAL SEARCH REPORT**

International application No. **PCT/CN2023/073612**

**A. CLASSIFICATION OF SUBJECT MATTER**

C07D471/04(2006.01)i;C07D247/02(2006.01)i;C07D309/16(2006.01)i;A61K31/437(2006.01)i;A61K31/506(2006.01)i; A61K31/341(2006.01)i;A61P19/10(2006.01)i;A61P35/00(2006.01)i;A61P9/00(2006.01)i

According to International Patent Classification (IPC) or to both national classification and IPC

**B. FIELDS SEARCHED**

Minimum documentation searched (classification system followed by classification symbols)

IPC:C07D,A61K,A61P

Documentation searched other than minimum documentation to the extent that such documents are included in the fields searched

Electronic data base consulted during the international search (name of data base and, where practicable, search terms used)

CNABS, DWPI, CNKI, Caplus(STN), Registry(STN): 组织蛋白酶K, 骨质疏松, 心血管, 牙龈炎, 嘧啶, 腈, 哌嗪, 四氢呋喃, 吲哚, 吡啶, cathepsin K, Cat K, osteoporosis, cardiovascular, gingivitis, pyrimidine, nitrile, piperazine, tetrahydrofuran, indole, pyridine

**C. DOCUMENTS CONSIDERED TO BE RELEVANT**

| Category* | Citation of document, with indication, where appropriate, of the relevant passages | Relevant to claim No. |
|---|---|---|
| X | 美国化学会 (American Chemical Society). "RN 1982714-07-7 etc." *Registry数据库 (REGISTRY Database),* 30 August 2016 (2016-08-30), pages 1-3 | 1 |
| X | CN 1678613 A (NOVARTIS AG) 05 October 2005 (2005-10-05) claims 1 and 2; and description, pages 15-18, 57, and 60 | 1, 2-12 (in part) |
| X | TENO, Naoki et al. "New Chemotypes for Cathepsin K Inhibitors" *Bioorganic & Medicinal Chemistry Letters,* Vol. 18, No. 8, 16 March 2008 (2008-03-16), pages 2599-2603, in particular page 2600, figure 2 | 1, 2-6 (in part), 10-12 (in part) |
| A | CN 101687864 A (ASTRAZENECA AB et al.) 31 March 2010 (2010-03-31) claim 1; and description, pages 26-29 | 2-12 (in part) |
| A | MILTZ, Wolfgang et al. "Design and Synthesis of Potent and Orally Active GPR4 Antagonists with Modulatory Effects on Nociception, Inflammation, and Angiogenesis" *Bioorganic & Medicinal Chemistry,* Vol. 25, No. 16, 29 June 2017 (2017-06-29), pages 4512-4525, in particular page 4513, table 1, and page 4514, tables 2b and 3 | 1-12 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **04 April 2023** | **19 April 2023** |
| Name and mailing address of the ISA/CN<br>**China National Intellectual Property Administration (ISA/CN)**<br>**China No. 6, Xitucheng Road, Jimenqiao, Haidian District, Beijing 100088**<br>Facsimile No. **(86-10)62019451** | Authorized officer<br><br>Telephone No. |

Form PCT/ISA/210 (second sheet) (July 2022)

**INTERNATIONAL SEARCH REPORT**

International application No.
**PCT/CN2023/073612**

**C. DOCUMENTS CONSIDERED TO BE RELEVANT**

| Category* | Citation of document, with indication, where appropriate, of the relevant passages | Relevant to claim No. |
|---|---|---|
| A | JP H05194418 A (KIRIN BREWERY) 03 August 1993 (1993-08-03)<br>claims 1 and 2 | 1-12 |
| A | CN 106478564 A (GUANGDONG HEC PHARMACEUTICAL CO., LTD.) 08 March 2017 (2017-03-08)<br>claims 1-10 | 1-12 |

Form PCT/ISA/210 (second sheet) (July 2022)

**INTERNATIONAL SEARCH REPORT**
**Information on patent family members**

International application No.
**PCT/CN2023/073612**

| Patent document cited in search report | Publication date (day/month/year) | Patent family member(s) | Publication date (day/month/year) |
|---|---|---|---|
| CN 1678613 A | 05 October 2005 | US 2009227596 A1 | 10 September 2009 |
| | | US 7820818 B2 | 26 October 2010 |
| | | US 2006142575 A1 | 29 June 2006 |
| | | US 7544688 B2 | 09 June 2009 |
| | | JP 2006500385 A | 05 January 2006 |
| | | GB 0220187 D0 | 09 October 2002 |
| | | DE 60313624 D1 | 14 June 2007 |
| | | DE 60313624 T2 | 27 December 2007 |
| | | PT 1537111 E | 09 August 2007 |
| | | HK 1078855 A1 | 24 March 2006 |
| | | BR 0313968 A | 19 July 2005 |
| | | AT 361300 T | 15 May 2007 |
| | | ES 2285239 T3 | 16 November 2007 |
| | | EP 1537111 A1 | 08 June 2005 |
| | | EP 1537111 B1 | 02 May 2007 |
| | | WO 2004020441 A1 | 11 March 2004 |
| | | CA 2494931 A1 | 11 March 2004 |
| | | AU 2003266330 A1 | 19 March 2004 |
| CN 101687864 A | 31 March 2010 | JP 2010531344 A | 24 September 2010 |
| | | CL 2008001899 A1 | 17 July 2009 |
| | | WO 2009001129 A1 | 31 December 2008 |
| | | WO 2009001129 A9 | 04 February 2010 |
| | | KR 20100039862 A | 16 April 2010 |
| | | BRPI 0813306 A2 | 16 May 2017 |
| | | CO 6251264 A2 | 21 February 2011 |
| | | NZ 581719 A | 26 August 2011 |
| | | EP 2170879 A1 | 07 April 2010 |
| | | EP 2170879 B1 | 16 January 2013 |
| | | ECSP 109882 A | 26 February 2010 |
| | | US 2009012077 A1 | 08 January 2009 |
| | | US 8008279 B2 | 30 August 2011 |
| | | IL 202519 A0 | 30 June 2010 |
| | | PE 20090841 A1 | 02 August 2009 |
| | | RU 2010101279 A | 10 August 2011 |
| | | RU 2470023 C2 | 20 December 2012 |
| | | CA 2689945 A1 | 31 December 2008 |
| | | TW 200911806 A | 16 March 2009 |
| | | UY 31180 A1 | 30 January 2009 |
| | | AR 067156 A1 | 30 September 2009 |
| | | AU 2008269513 A1 | 31 December 2008 |
| | | AU 2008269513 B2 | 16 June 2011 |
| JP H05194418 A | 03 August 1993 | None | |
| CN 106478564 A | 08 March 2017 | None | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **GARG G** ; **PRADEEP AR** ; **THORAT MK et al.** Effect of nonsurgical periodontal therapy on crevicular fluid levels of cathepsin K in periodontitis. *Arch Oral Biol*, vol. 54, 1046-1051
- **SAULNIER, M.G. et al.** *Bioorg. Med. Chem. Lett*, 1994, vol. 4, 1985-1990
- **GREENWALD, R.B et al.** *J.Med.Chem.*, 2000, vol. 43, 475